(19) Europäisches Patentamt
European Patent Office
Office européen des brevets

(11) Numéro de publication: **0 205 375**
**B1**

(12) # FASCICULE DE BREVET EUROPEEN

(45) Date de publication du fascicule du brevet: **08.11.89**

(51) Int. Cl.⁴: **C 07 D 215/22**, C 07 D 401/12, C 07 D 409/04, C 07 D 401/04, A 61 K 31/47

(21) Numéro de dépôt: **86401141.6**

(22) Date de dépôt: **29.05.86**

(54) **Amides dérivés de la quinoléine, procédés pour leur préparation et médicaments les contenant.**

(30) Priorité: **30.05.85 FR 8508111**

(43) Date de publication de la demande: **17.12.86 Bulletin 86/51**

(45) Mention de la délivrance du brevet: **08.11.89 Bulletin 89/45**

(84) Etats contractants désignés: **AT BE CH DE FR GB IT LI LU NL SE**

(56) Documents cités:
**EP-A- 0 112 776**
**GB-A- 1 064 252**

**Le dossier contient des informations techniques présentées postérieurement au dépôt de la demande et ne figurant pas dans le présent fascicule.**

(73) Titulaire: **RHONE-POULENC SANTE, 20, avenue Raymond Aron, F-92160 Antony (FR)**

(72) Inventeur: **Benavides, Jesus, 47 Avenue de Seine, F-92500 Rueil-Malmaison (FR)**
Inventeur: **Dubroeucq, Marie Christine, 13 Villa Malleville, F-95880 Enghien Les Bains (FR)**
Inventeur: **Le Fur, Gérard, 19 ter rue des Carrières, F-95160 Montmorency (FR)**
Inventeur: **Renault, Christian, 61 rue des Mallets, F-95150 Taverny (FR)**

(74) Mandataire: **Savina, Jacques et al, RHONE-POULENC INTERSERVICES Service Brevets Pharma 25, quai Paul Doumer, F-92408 Courbevoie Cédex (FR)**

EP 0 205 375 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

**Description**

La présente invention a pour objet des amides dérivés de la quinoléine, leur procédés de préparation et les médicaments les contenant.

Des dérivés de la quinoléine ayant une activité anxiolytique sont décrits dans le brevet EP 112 76.

Les composés selon l'invention peuvent être représentés par la formule générale:

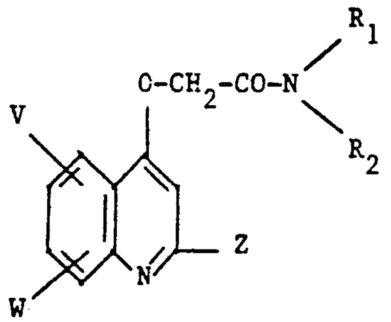

(I)

dans laquelle,

V et W, identiques ou différents, représentent des atomes d'hydrogène ou d'halogène (fluor, chlore, brome), des groupes alkyle comportant 1 à 4 atomes de carbone, alcoxy comportant 1 à 4 atomes de carbone, amino ou acylamino comportant 1 à 4 atomes de carbone,

Z représente un radical phényle, thiényle, pyridyle ou phényle substitué par un ou deux substituants pris parmi les atomes d'halogène, les groupes alkyle et alcoxy comportant 1 à 4 atomes de carbone, les groupes trifluorométhyle, nitro ou amino,

$R_1$ représente un groupe alkyle linéaire ou ramifié comportant 1 à 6 atomes de carbone, alcoxyalkyle dont les parties alcoxy et alkyle comportent chacune 1 à 4 atomes de carbone, cycloalkyle comportant 3 à 6 atomes de carbone, phényle, phénylalkyle dont la partie alkyle comporte 1 à 4 atomes de carbone ou cycloalkylalkyle dont la partie alkyle comporte 1 à 3 atomes de carbone et la partie cycloalkyle comporte 3 à 6 atomes de carbone,

$R_2$ représente un groupe alkyle linéaire ou ramifié comportant 1 à 6 atomes de carbone, alcoxyalkyle dont les parties alcoxy et alkyle comportent chacune 1 à 4 atomes de carbone, cycloalkyle comportant 3 à 6 atomes de carbone, phényle, phénylalkyle dont la partie alkyle comporte 1 à 4 atomes de carbone ou cycloalkylalkyle dont la partie alkyle comporte 1 à 3 atomes de carbone et la partie cycloalkyle comporte 3 à 6 atomes de carbone ou un cycle morpholine-4,

$R_1$ et $R_2$ peuvent également former ensemble avec l'atome d'azote auquel ils sont rattachés un cycle pyrrolidine, pipéridine éventuellement substitué par un groupe hydroxy, alkyle comportant 1 à 4 atomes de carbone, alcoxy comportant 1 à 4 atomes de carbone, alkyloxycarbonyle dont la partie alkyle comporte 1 à 4 atomes de carbone, un cycle morpholine éventuellement substitué par un ou deux groupes alkyle comportant 1 à 4 atomes de carbone, un cycle thiomorpholine, un cycle pipérazine éventuellement substitué sur l'atome d'azote par un groupe alkyle comportant 1 à 4 atomes de carbone, alkyloxycarbonyle dont la partie alkyle comporte 1 à 4 atomes de carbone, acyle comportant 2 à 5 atomes de carbone, formyle ou un cycle pipérazinone éventuellement substitué sur l'atome d'azote par un groupe alkyle comportant 1 à 4 atomes de carbone.

Lorsque les groupes $R_1$ et $R_2$ comportent un ou plusieurs atomes de carbone asymétriques, il y a plusieurs stéréoisomères correspondant à la formule plane (I). Ces divers stéréoisomères font également partie de l'invention.

Les composés de formule (I) peuvent être préparés par action d'une amine de formule:

$$HN\begin{matrix} R_1 \\ R_2 \end{matrix} \quad (II)$$

dans laquelle $R_1$ et $R_2$ ont les mêmes significations que dans la formule (I) ou un sel de cette amine, sur un dérivé de formule:

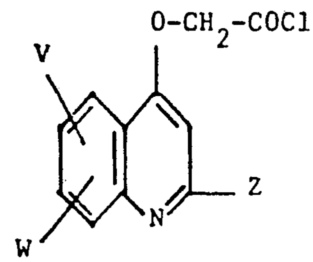

(III)

dans laquelle V, W et Z ont les mêmes significations que dans la formule (I).

Comme sel de l'amine de formule (II) on peut citer par exemple le chlorhydrate ou le paratoluènesulfonate.

Cette réaction peut être réalisée selon des procédés connus en soi, permettant de transformer un chlorure d'acide carboxylique en carboxamide tels que ceux décrits par C.A. BUEHLER et D.E. PEARSON, Survey of Organic Synthesis, Wiley interscience, p. 894, 1970.

On peut traiter le chlorure d'acide de formule (III) par un excès de l'amine de formule (II) au sein d'un solvant inerte tel que le toluène, le chloroforme ou le chlorure de méthylène à une température comprise entre 20°C et la température d'ébullition du solvant utilisé. L'excès d'amine utilisé, qui joue le rôle de base neutralisant l'acide chlorhydrique formé dans la réaction est d'au moins un équivalent c'est-à-dire que la quantité totale d'amine employée est au moins de deux équivalents.

On peut aussi faire réagir le chlorure d'acide de formule (III) avec l'amine de formule (II) en présence d'une amine tertiaire telle que la triéthylamine, au sein d'un solvant inerte tel que le toluène, le chloroforme ou le chlorure de méthylène, à une température comprise entre 20°C et la température d'ébullition du solvant. Dans le cas où l'on utilise un sel d'amine de formule (II), il est nécessaire d'utiliser au moins 2 équivalents d'amine tertiaire pour un équivalent de sel d'amine.

Dans le cas où le chlorure d'acide de formule (III) est sous forme de chlorhydrate, il est nécessaire d'utiliser un équivalent supplémentaire d'amine de formule (II) ou d'amine tertiaire.

Les composés de formule (III) peuvent être obtenus par action sur un acide de formule:

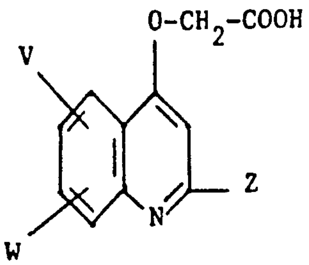

(IV)

dans laquelle V, W et Z ont les mêmes significations que dans la formule (III) d'un agent de chloruration tel que le chlorure de thionyle.

La réaction de l'acide de formule (IV) avec l'agent de chloruration peut être réalisée en l'absence de solvant ou au sein d'un solvant inerte tel que le chloroforme ou le toluène, de préférence à la température de reflux du milieu.

Les acides de formule (IV) peuvent être obtenus par application ou adaptation des méthodes décrites dans l'exemple 1 et dans R.B. WAGNER et H.D. ZOOK, Synthetic Organic Chemistry, J. Wiley, p. 411-478 (1953, C.A. BUEHLER et D.E. PEARSON, Survey of organic synthesis, Wiley interscience, p. 655-710 (1970).

Les composés de formule (I) dans laquelle Vet W, identiques ou différents, représentent des atomes d'hydrogène ou d'halogène, des groupes alkyle comportant 1 à 4 atomes de carbone ou alcoxy comportant 1 à 4 atomes de carbone, Z représente un radical phényle, thiényle, pyridyle ou phényle substitué par un ou deux substituants pris parmi les atomes d'halogène, les groupes alkyle et alcoxy comportant 1 à 4 atomes de carbone, des groupes trifluorométhyle ou nitro, $R_1$ et $R_2$ sont définis comme précédemment peuvent être aussi préparés par action d'un composé de formule:

$$Hal\text{-}CH_2\text{-}CO\text{-}N\begin{smallmatrix}R_1\\R_2\end{smallmatrix} \quad (V)$$

dans laquelle $R_1$ et $R_2$ ont les mêmes significations que précédemment, Hal représente un atome d'halogène (chlore ou brome) sur un dérivé de formule:

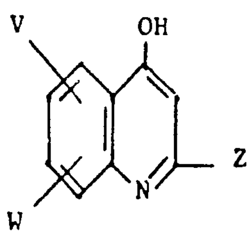

(VI)

dans laquelle V, W et Z ont les mêmes significations que ci-dessus.

Cette réaction peut être réalisée selon des procédés connus comme celui décrit dans Chem. Abst 95, 203 770K (1981) qui consiste à opérer en présence d'une base telle que le carbonate de potassium, de préférence en présence d'iodure cuivreux, au sein d'un solvant tel que la butanone-2 et à une température comprise entre 20°C et la température d'ébullition du solvant.

La plupart des composés de formule (VI) sont connus; les composés nouveaux peuvent être obtenus par application ou adaptation des méthodes décrites par C. HAUSER et A. REYNOLDS, J.A.C.S., 70, 2402 à 2404 (1948), A. KASAHARA, Chem. Ind. 4, 121 (1981), SORM, Chem. Listy, 49, 901 (1954), B. STASKUN et al., J. Org. Chem., 26, 319 (1961), et ELDERFIELD et al., J. Amer. Chem. Soc., 68, 1272 (1946).

Les composés de formule (I) dans laquelle V et W, identiques ou différents, représentent des atomes d'hydrogène ou d'halogène, des groupes alkyle comportant 1 à 4 atomes de carbone ou alcoxy comportant 1 à 4 atomes de carbone, Z représente un radical phényle, thiényle, pyridyle ou phényle substitué par un ou deux substituants pris parmi les atomes d'halogène, les groupes alkyle et alcoxy comportant 1 à 4 atomes de carbone, les groupes trifluorométhyle ou nitro et $NR_1R_2$ représente un cycle pipéridine substitué par un groupe alcoxy comportant 1 à 4 atomes de carbone peuvent aussi être préparés par action d'un composé de formule:

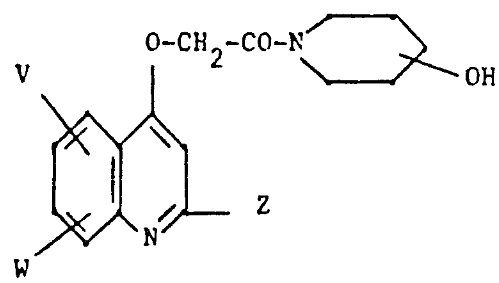

(VII)

dans laquelle V, W et Z ont les significations mentionnées ci-dessus sur un dérivé de formule:

$$Hal\text{-}R \qquad (VIII)$$

dans laquelle Hal représente un atome d'halogène (chlore ou brome) et R représente un groupe alkyle comportant 1 à 4 atomes de carbone.

Cette réaction est effectuée dans un solvant inerte tel que le tétrahydrofuranne en présence d'une base telle que l'hydrure de sodium à la température d'ébullition du solvant.

Les composés de formule (I) dans laquelle V et W, identiques ou différents représentent des atomes d'hydrogène ou d'halogène, des groupes alkyle comportant 1 à 4 atomes de carbone ou alcoxy comportant 1 à 4 atomes de carbone, Z représente un radical phényle, thiényle, pyridyle ou phényle substitué par un ou deux substituants pris parmi les atomes d'halogène, les groupes alkyle et alcoxy comportant 1 à 4 atomes de carbone, les groupes trifluorométhyle ou nitro et $NR_1R_2$ représente un cycle pipérazinone substitué sur l'atome d'azote par un groupe alkyle comportant 1 à 4 atomes de carbone peuvent également être préparés par alkylation des composés correspondants de formule (I) pour lesquels $NR_1R_2$ représente un cycle pipérazinone avec un halaogé-

nure d'alkyle de formule (VIII) dans laquelle Hal représente un atome d'halogène et R représente un groupe alkyle ayant 1 à 4 atomes de carbone.

Cette réaction peut être effectuée au sein d'un solvant inerte tel que le toluène, le diméthylformamide et le diméthylsulfoxyde, à la température ambiante, en présence d'une base telle que l'hydrure de sodium.

Les composés de formule (I) dans laquelle V et/ou W sont des groupes amino et/ou Z est un radical phényle substitué par un ou deux groupes amino, $R_1$ et $R_2$ sont définis comme précédemment, étant entendu que ces composés sont exempts de groupe nitro, peuvent être préparés par hydrogénation catalytique des dérivés nitrés correspondants. Cette réaction peut être effectuée au sein du méthanol à une température variant entre 20 et 40°C, en présence d'un catalyseur tel que le palladium sur charbon, sous une pression d'hydrogène de 1 bar.

Les composés de formule (I) dans laquelle V et/ou W sont des groupes amino et/ou Z est un radical phényle substitué par un ou deux groupes amino, $R_1$ et $R_2$ sont définis comme précédemment, étant entendu que ces composés sont exempts de groupe nitro peuvent être préparés par réduction des dérivés nitrés correspondants.

Cette réduction est avantageusement effectuée au sein de l'éthanol au moyen d'une solution aqueuse de dithionite de sodium, à la température d'ébullition du solvant.

Les composés de formule (I) dans laquelle V et/ou W sont des groupes acylamino comportant 1 à 4 atomes de carbone, Z, $R_1$ et $R_2$ sont définis comme précédemment, étant entendu que Z n'est pas un radical phényle substitué par un groupe amino, peuvent être obtenus par acylation des dérivés aminés correspondants de formule (I) au moyen d'agent d'acylation de formule:

Hal-CO-E (IX)

dans laquelle Hal représente un atome d'halogène et E représente un atome d'hydrogène ou un groupe alkyle ayant 1 à 3 atomes de carbone.

Cette réaction est avantageusement effectuée au sein du chlorure de méthylène en présence d'une base telle que la triéthylamine.

Les énantiomères des composés de formule (I) peuvent être obtenus par dédoublement des racémiques, par exemple, par chromatographie sur colonne chirale selon W.H. PIRKLE et Coll., asymetric synthesis, vol. 1, Academic Press (1983) ou encore par synthèse à partir de précurseurs chiraux.

Les mélanges réactionnels obtenus par les divers procédés décrits précédemment sont traités suivant des méthodes classiques physiques (évaporation, extraction, distillation, cristallisation, chromatographie) ou chimiques le cas échéant (formation de sel et régéneration de la base ou de l'acide) afin d'isoler les composés de formule (I) à l'état pur.

Lorsque cela est possible, les composés de formule (I) sous forme de base libre peuvent être éventuellement transformés en sels d'addition avec un acide minéral ou organique par action d'un tel acide au sein d'un solvant organique tel qu'un alcool, une cétone, un éther ou un solvant chloré.

Les composés de formule (I) et leurs sels présentent des propriétés pharmacologiques intéressantes. Ces composés se lient aux récepteurs des benzodiazépines du type cérébral et sont donc utiles comme anxiolytiques.

L'affinité des composés de formule (I) pour les récepteurs de type cérébral des benzodiazépines a été déterminée selon la méthode de MOHLER et Coll., Life Sciences, 20, 2101 (1977) sur des membranes de cerveau de rat en utilisant comme ligand le 3H-diazepam. Cette affinité est comprise entre 0,001 et 0,5 µM.

Les composés selon l'invention présentent une faible toxicité. Leur $DL_{50}$ par voie orale chez la souris est supérieure à 200 mg/kg. Les $DL_{50}$ ont été calculées après 3 jours d'observation par la méthode cumulative de J.J. REED et H.MUENCH, Amer. J. Hyg., 27, 493 (1938).

Sont particulièrement intéressants les composés suivants:

- N-isopropyl N-méthyl (phényl-2 quinolyl-4) oxyacétamide
- ([(méthyl-4 phényl)-2 quinolyl-4] oxyacétyl)-4 morpholine
- ([(fluoro-2 phényl)-2 quinolyl-4] oxyacétyl)-4 morpholine
- ([(thiényl-2)-2 quinolyl-4] oxyacétyl)-4 morpholine
- [(-phényl-2 quinolyl-4) oxyacétyl]-4 thiomorpholine
- N-cyclopropylméthyl N-méthyl (phényl-2 quinolyl-4) oxyacétamide
- ([(chloro-3 phényl)-2 quinolyl-4] oxyacétyl)-4 morpholine
- Diméthyl-2,6 [(phényl-2 quinolyl-4) oxyacétyl]-4 morpholine
- [(phényl-2 quinolyl-4) oxyacétyl]-1 pipéridinol-4
- [(phényl-2 quinolyl-4) oxyacétyl]-4 pipérazinone-2
- formyl-1 [(phényl-2 quinolyl-4) oxyacétyl]-4 pipérazine
- [(chloro-5 phényl-2 quinolyl-4) oxyacétyl]- morpholine
- N-méthyl N-morpholinyl-4 (phényl-2 quinolyl-4) oxyacétamide
- [(chloro-6 phényl-2 quinolyl-4) oxyacétyl]-4 morpholine
- [(phényl-2 quinolyl-4) oxyacétyl]-1 pyrrolidine
- N,N-diéthyl (phényl-2 quinolyl-4) oxyacétamide
- [(phényl-2 quinolyl-4) oxyacétyl]-1 pipéridine
- [(phényl-2 quinolyl-4) oxyacétyl]-1 morpholine
- N-méthyl N-(méthyl-1 propyl) (phényl-2 quinolyl-4) oxyacétamide
- ([(-chloro-4 phényl-2) quinolyl-4] oxyacétyl)-4 morpholine
- [(méthyl-6 phényl-2 quinolyl-4) oxyacétyl]-4 morpholine
- méthyl-3 [(phényl-2 quinolyl-4) oxyacétyl]-1 pipéridine
- [(acétylamino-6 phényl-2 quinolyl-4) oxyacétyl]-4 morpholine
- ([(amino-4 phényl)-2 quinolyl-4] oxyacétyl)-4 morpholine
- ([(thiényl-3)-2 quinolyl-4] oxyacétyl)-4 morpholine
- N-cyclobutyl N-méthyl (phényl-2 quinolyl-4) oxyacétamide

Pour l'emploi médicinal, il peut être fait usage des produits de formule (I) tels quels ou lorsqu'ils peuvent exister, à l'état de sels avec un acide fort pharmaceutiquement acceptable.

Comme sels pharmaceutiquement acceptables, on peut citer les sels d'addition avec les acides minéraux tels que chlorhydrates, sulfates, nitrates, phosphates ou organiques tels que acétates, propionates, succinates, benzoates, fumarates, théophilline-acétates, salicylates, méthylènebis-β-oxynaphtoates ou des dérivés de substitution de ces produits.

Les exemples suivants montrent comment l'invention peut être mise en pratique.

*Exemple 1*

On chauffe 8 heures 30 minutes au reflux 3,5 g d'acide (phényl-2 quinolyl-4)oxyacétique et 2,8 $cm^3$ de chlorure de thionyle dans 300 $cm^3$ de chloroforme. On élimine les solvants sous pression réduite, et le résidu obtenu est ajouté, en 15 minutes, à une solution préalablement refroidie à 5°C de 13 $cm^3$ de diéthylamine dans 100 $cm^3$ de chlorure de méthylène. On agite 1 heure 30 minutes à 5-10°C, lave la phase organique par 6 fois 100 $cm^3$ d'eau, sèche sur sulfate de magnésium et évapore sous pression réduite. Le résidu obtenu est chromatographié sur gel de silice en utilisant comme éluant le mélange cyclohexane-acétate d'éthyle (80-20 en volume).

Le résidu obtenu est recristallisé dans un mélange éther isopropylique-acétonitrile (10-1 en volume). On isole ainsi 1,91 g de N,N-diéthyl [(phényl-2 quinolyl-4)oxy]-acétamide fondant à 97°C.

L'acide (phényl-2 quinolyl-4) oxyacétique peut être préparé par saponification de l'ester éthylique correspondant au moyen d'une solution normale d'hydroxyde de sodium. Il présente un point de fusion égal à 179-181°C.

Le (phényl-2 quinolyl-4) oxyacétate d'éthyle peut être préparé de la façon suivante:

A une suspension agitée de 8,8 g de phényl-2 quinolinol-4 et de 11 g de carbonate de potassium dans 300 $cm^3$ de méthyléthylcétone, on ajoute, goutte à goutte, 4,4 $cm^3$ de bromoacétate d'éthyle.

On chauffe 3 heures au reflux. On ramène à température ambiante (20°C environ), essore l'insoluble et élimine les solvants sous pression réduite.

Le résidu est repris par 100 $cm^3$ d'éther de pétrole 40°-70° et filtré. On isole ainsi 11,1 g de (phényl-2 quinolyl-4)oxyacétate d'éthyle fondant à 96°C.

Le phényl-2 quinolinol-4 peut être préparé selon C. HAUSER et A. REYNOLDS, J.A.C.S., 70, 2402 (1948).

*Exemple 2*

On opère comme à l'exemple 1 mais en partant de 3 g d'acide (phényl-2 quinolyl-4)oxyacétique de 2,35 $cm^3$ de chlorure de thionyle dans 50 $cm^3$ de chloroforme, de 1,04 $cm^3$ de pipéridine et de 3 $cm^3$ de triéthylamine dans 50 $cm^3$ de toluène. Le résidu obtenu est transformé au sein de l'acétone en son chlorhydrate par addition d'une solution d'acide chlorhydrique dans l'éther éthylique. Après recristallisation dans l'éthanol, on obtient 0,46 g de chlorhydrate de [(phényl-2 quinolyl-4)oxyacétyl]-1 pipéridine fondant à 146°C.

*Exemple 3*

On opère comme à l'exemple 1 mais en partant de 3 g d'acide (phényl-2 quinolyl-4)oxyacétique, de 2,35 $cm^3$ de chlorure de thionyle dans 80 $cm^3$ de chloroforme, de 1,18 $cm^3$ de N-méthylpipérazine et de 3 $cm^3$ de triéthylamine dans 50 $cm^3$ de toluène.

Le résidu obtenu est repris dans l'acétone et après addition d'une solution d'acide chlorhydrique dans l'éther éthylique, on isole 1,1 g de dichlorhydrate de méthyl-4 [(phényl-2 quinolyl-4)oxyacétyl]-1 pipérazine fondant à 182°C.

*Exemple 4*

On opère comme à l'exemple 1 mais en partant de 3 g d'acide (phényl-2 quinolyl-4)oxyacétique, de 3,12 $cm^3$ de chlorure de thionyle dans 100 $cm^3$ de chloroforme, de 0,94 $cm^3$ de morpholine et de 3 $cm^3$ de triéthylamine dans 50 $cm^3$ de toluène.

Le résidu obtenu est recristallisé deux fois dans l'acétate d'éthyle. On obtient ainsi 1,43 g de [(phényl-2 quinolyl-4)-oxyacétyl]-4 morpholine fondant à 145°C.

*Exemple 5*

A une suspension agitée de 3,5 g de (chloro-4 phényl)-2 hydroxy-4 quinoléine et de 3,74 g de carbonate de potassium anhydre dans 200 $cm^3$ de butanone-2, on ajoute une solution de 2,94 g de (bromo-2 acétyl)-4 morpholine dans 40 $cm^3$ de méthyléthylacétone.

On chauffe au reflux 15 heures, refroidit à température ambiante (20°C environ), élimine l'insoluble par filtration, et évapore la méthyléthylacétone sous pression réduite.

Après recristallisation du résidu dans un mélange éther isopropylique-acétate d'éthyle (10-1 en volume), on isole 1,6 g de [(chloro-4 phényl)-2 quinolyl-4)oxyacétyl]-4 morpholine que l'on transforme au sein de l'acétone en son chlorhydrate par addition d'une solution d'acide chlorhydrique dans l'éther éthylique. Ce chlorhydrate présente un point de fusion de 191°C.

La (chloro-4 phényl)-2 hydroxy-4 quinoléine peut être préparée selon KASAHARA et al., Chem. Ind (London) (4), 121 (1981).

*Exemple 6*

On opère comme à l'exemple 1 mais en partant de 3 g d'acide (phényl-2 quinolyl-4)oxyacétique, de 3,12 $cm^3$ de chlorure de thionyle dans 100 $cm^3$ de triéthylamine de 1,39 g de N-méthyl butanamine-2 et de 3 $cm^3$ de triéthylamine dans 50 $cm^3$ de toluène sur tamis. Le résidu obtenu est repris dans l'acétone, et après addition d'une solution d'acide chlorhydrique dans l'éther éthylique et 2 recristallisations, la première dans un mélange éthanol-éther éthylique, la seconde dans l'isopropanol, on isole 0,69 g de chlorhydrate de N-méthyl N-(méthyl-1 propyl) (phényl-2 quinolyl-4) oxyacétamide fondant à 172°C.

*Exemple 7*

On opère comme à l'exemple 5, en partant de 3 g de (méthoxy-4 phényl)-2 hydroxy-4 quinoléine, de 2,6 g de (bromo-2 acétyl)-4 morpholine, de 3,3 g de

carbonate de potassium dans 100 $cm^3$ de méthyléthylacétone. Après chromatographie du résidu sur du gel de silice en utilisant comme éluant l'acétate d'éthyle, le produit obtenu est repris dans l'acétone et après addition d'une solution d'acide chlorhydrique dans l'éther éthylique, on isole 1,2 g de chlorhydrate de ([(méthoxy-4 phényl)-2 quinolyl-4] oxyacétyl)-4 morpholine fondant à 215°C.

La (méthoxy-4 phényl)-2 hydroxy-4 quinoléine peut être préparée selon SORM, Chem. Listy 49, 901 (1954).

*Exemple 8*

On chauffe 3 heures au reflux 1,8 g d'acide (phényl-2 quinolyl-4) oxyacétique et 1,41 $cm^3$ de chlorure de thionyle dans 54 $cm^3$ de chloroforme. On élimine le solvant sous pression réduite et le résidu obtenu est mis en suspension dans 40 $cm^3$ de chloroforme.

A cette suspension, on ajoute lentement, sous agitation, 0,83 $cm^3$ de N-méthylbenzylamine et 2 $cm^3$ de triéthylamine dans 10 $cm^3$ de chloroforme, en maintenant la température à 10°C. On agite 15 minutes à température ambiante (environ 20°C), évapore le solvant sous pression réduite et reprend le résidu par 50 $cm^3$ d'acétate d'éthyle et 20 $cm^3$ d'eau. La phase organique est décantée, lavée par 2 fois 10 $cm^3$ d'eau puis par 10 $cm^3$ d'une solution normale d'acide chlorhydrique et enfin par 10 $cm^3$ d'eau.

Après évaporation du solvant sous pression réduite, le résidu est dissous dans l'acétonitrile et cristallisé par addition lente d'éther isopropylique. Après recristallisation du produit brut dans un mélange acétonitrile-éther isopropylique, on isole 0,85 g de N-benzyl N-méthyl (phényl-2 quinolyl-4) oxyacétamide fondant à 102°C.

*Exemple 9*

On opère comme à l'exemple 1 à partir de 6 g d'acide (phényl-2 quinolyl-4) oxyacétique, de 4,6 $cm^3$ de chlorure de thionyle dans 180 $cm^3$ de chloroforme, de 3,31 g de pipérazine-1 carboxylate d'éthyle et de 6,5 $cm^3$ de triéthylamine dans 200 $cm^3$ de chloroforme.

Après recristallisation du résidu dans un mélange acétate d'éthyle-éthanol (5-1 en volume), on isole 3 g de [(phényl-2 quinolyl-4) oxyacétyl]-4 pipérazine-1 carboxylate d'éthyle fondant à 163°C.

*Exemple 10*

On opère comme à l'exemple 1 à partir de 2 g d'acide (phényl-2 quinolyl-4)oxyacétique, de 1,5 $cm^3$ de chlorure de thionyle dans 70 $cm^3$ de chloroforme, de 0,71 g de thiomorpholine et de 2,1 $cm^3$ de triéthylamine dans 70 $cm^3$ de chloroforme.

Après dissolution dans l'acétate d'éthyle et cristallisation par addition d'éther isopropylique, on isole 1,8 g de [(phényl-2 quinolyl-4)oxy acétyl]-4 thiomorpholine fondant à 130°C.

*Exemple 11*

On opère comme à l'exemple 1 à partir de 2,2 g d'acide (chloro-4 phényl-2 quinolyl-4) oxyacétique, de 1,5 $cm^3$ de chlorure de thionyle dans 70 $cm^3$ de chloroforme, de 0,71 g de thiomorpholine et de 2,1 $cm^3$ de triéthylamine dans 70 $cm^3$ de chloroforme.

Après recristallisation dans l'acétate d'éthyle, on isole 0,8 g de ([(chloro-4 phényl)-2 quinolyl-4] oxyacétyl)-4 thiomorpholine fondant à 117°C.

L'acide (chloro-4 phényl-2 quinolyl-4) oxyacétique est préparé de la même manière que l'acide (phényl-2 quinolyl-4) oxyacétique décrit dans l'exemple 1. Son point de fusion est de 230°C avec décomposition.

*Exemple 12*

On opère comme à l'exemple 5 à partir de 3 g de méthoxy-6 phényl-2 quinolinol-4, de 2,3 g (bromo-2 acétyl)-4 morpholine, de 3,3 g de carbonate de potassium dans 100 $cm^3$ de méthyléthylacétone et en réduisant à 6 heures le temps de réaction.

Le résidu est transformé au sein d'un mélange éther éthylique-acétone (5-1 en volume) en un chlorhydrate brut par addition d'une solution d'acide chlorhydrique dans l'isopropanol. Ce chlorhydrate est repris à chaud dans un mélange acétone-eau. Après filtration et séchage, on obtient 2,2 g de chlorhydrate de [(méthoxy-6 phényl-2 quinolyl-4) oxyacétyl]-4 morpholine fondant à 179°C.

Le méthoxy-6 phényl-2 quinolinol-4 peut être préparé selon B. STASKUN et S.S. ISRAELSTAM, J. Org. Chem., (26), p. 3191 (1961).

*Exemple 13*

On opère comme à l'exemple 1 à partir de 3 g d'acide (phényl-2 quinolyl-4) oxyacétique, de 3,8 $cm^3$ de chlorure de thionyle dans 90 $cm^3$ de chloroforme, de 12,5 $cm^3$ d'une solution 3M de diméthylamine dans le toluène, et en réduisant à 3 heures le temps de préparation du chlorure d'acide.

Après recristallisation du résidu dans un mélange toluène-éther isopropylique, on isole 2,2 g de N,N-diméthyl (phényl-2 quinolyl-4) oxyacétamide fondant à 112°C.

*Exemple 14*

On opère comme à l'exemple 1 à partir de 3 g d'acide (phényl-2 quinolyl-4) oxyacétique de 3,8 $cm^3$ de chlorure de thionyle dans 90 $cm^3$ de chloroforme et de 2,7 $cm^3$ de N-méthyl isopropylamine dans 90 $cm^3$ de chloroforme, et en réduisant à 3 heures les temps de préparation du chlorure d'acide.

Après chromatographie du résidu sur du gel de silice au moyen d'un éluant cyclohexane-acétate d'éthyle (50-50 en volume), et recristallisation dans l'éther isopropylique, on isole 1,3 g de N-méthyl N-isopropyl (phényl-2 quinolyl-4)oxyacétamide fondant à 80°C.

*Exemple 15*

On opère comme à l'exemple 1 à partir de 3 g d'acide (phényl-2 quinolyl-4) oxyacétique, de 3,8 $cm^3$ de chlorure de thionyle dans 90 $cm^3$ de chloroforme et de 1,32 $cm^3$ de diméthyl-2,6 morpholine et 3,25 $cm^3$ de triéthylamine dans 90 $cm^3$ de chloroforme et en réduisant à 3 heures le temps de préparation du chlorure d'acide.

Après chromatographie du résidu sur du gel de silice au moyen d'un mélange cyclohexane-acétate d'éthyle comme éluant et recristallisation dans

l'éther isopropylique, on isole 1,7 g de diméthyl-2,6 [(phényl-2 quinolyl-4) oxyacétyl]-4 morpholine fondant à 117°C.

*Exemple 16*

On opère comme à l'exemple 1 à partir de 3 g d'acide (phényl-2 quinolyl-4) oxyacétique, de 3,8 $cm^3$ de chlorure de thionyle dans 90 $cm^3$ de chloroforme et de 1,08 g d'hydroxy-4 pipéridine et 3,25 $cm^3$ de triéthylamine dans 90 $cm^3$ de chloroforme et en réduisant à 3 heures le temps de préparation d'acide.

Le résidu est repris dans l'éthanol, et après addition d'une solution d'acide chlorhydrique dans l'éther éthylique, on isole 3 g de chlorhydrate de [(phényl-2 quinolyl-4)oxyacétyl]-1 pipéridinol-4 fondant à 185°C.

*Exemple 17*

On opère comme à l'exemple 1 à partir de 3 g d'acide (phényl-2 quinolyl-4)oxyacétique, de 3,8 $cm^3$ de chlorure de thionyle dans 90 $cm^3$ de chloroforme, de 1,34 g de chlorhydrate de N-méthyl méthoxy-2 éthylamine, de 4,5 $cm^3$ de triéthylamine dans 90 $cm^3$ de chloroforme, et en réduisant à 3 heures le temps de préparation du chlorure d'acide.

Après deux recristallisations dans un mélange acétate d'éthyle-éther isopropylique, on isole 1,6 g de N-méthyl N-(méthoxy-2 éthyl) (phényl-2 quinolyl-4) oxyacétamide fondant à 109°C.

La N-méthyl méthoxy-2 éthylamine peut être préparée selon MNDSHOJAN et al., DOKLADY AKAD. ARMJANSK, SSR 27 (1958) 161, 167.

*Exemple 18*

On opère comme à l'exemple 1 à partir de 3 g d'acide (phényl-2 quinolyl-4) oxyacétique, de 3,8 $cm^3$ de chlorure de thionyle dans 90 $cm^3$ de chloroforme, de 3,2 g de paratoluènesulfonate d'acétyl-1 pipérazine, de 4,5 $cm^3$ de triéthylamine dans 90 $cm^3$ de chloroforme et en réduisant à 3 heures le temps de préparation du chlorure d'acide.

Après chromatographie du résidu sur du gel de silice au moyen d'un mélange acétate d'éthyle-chloroforme-éthanol (70-20-10 en volume) comme éluant et recristallisation dans un mélange éthanol-éther isopropylique, on isole 1 g d'acétyl-1 (phényl-2 quinolyl-4) oxyacétyl]-4 pipérazine fondant à 184°C.

Le paratoluène sulfonate d'acétyl-1 pipérazine peut être préparé selon H.K. HALL, J. Am. Soc., (78), 2570 (1956).

*Exemple 19*

On opère comme à l'exemple 1 à partir de 3 g d'acide (phényl-2 quinolyl-4) oxyacétique, de 3,8 $cm^3$ de chlorure de thionyle dans 90 $cm^3$ de chloroforme, de 1,07 g de pipérazinone-2 et 3,25 $cm^3$ de triéthylamine dans 90 $cm^3$ de chloroforme, et en réduisant à 3 heures le temps de préparation du chlorure d'acide.

En fin de réaction, on essore le précipité, le lave à l'eau, puis par une solution décinormale d'hydroxyde de sodium, puis encore à l'eau; on recristallise le précipité dans l'acide acétique.

On obtient ainsi 1,9 g de [(phényl-2 quinolyl-4) oxyacétyl]-4 pipérazinone-2 fondant à 215°C.

La pipérazinone-2 peut être préparée selon S.R. ASPINALL, J. Am. Chem. Soc. (62), 1202 (1940).

*Exemple 20*

On place sous atmosphère d'azote, 3,8 g [(phényl-2 quinolyl-4) oxyacétyl]-1 pipéridinol-4 préparé selon l'exemple 16 dans 80 $cm^3$ de tétrahydrofuranne sec.

On ajoute peu à peu 0,42 g d'hydrure de sodium en dispersion à 60% dans l'huile, puis agite 2 heures température ambiante (20°C environ). On chauffe ensuite au reflux, puis coule lentement en 30 minutes 1,37 $cm^3$ d'iodure de méthyle dans 10 $cm^3$ de tétrahydrofuranne. On chauffe une heure au reflux, ajoute encore 1,37 $cm^3$ d'iodure de méthyle dans 100 $cm^3$ de tétrahydrofuranne et chauffe 30 minutes au reflux, refroidit à température ambiante et ajoute 0,21 g d'hydrure de sodium en dispersion à 60% dans l'huile puis chauffe au reflux 30 minutes. On réitère 2 fois la double addition d'iodure de méthyle et d'hydrure de sodium dans les conditions décrites ci-dessus.

Le solvant est évaporée sous pression réduite, le résidu repris par 150 $cm^3$ d'eau et 100 $cm^3$ d'acétate d'éthyle. La phase organique est lavée à l'eau, séchée sur sulfate de magnésium et évaporée sous pression réduite.

Le résidu est chromatographié sur du gel de silice en utilisant l'acétate d'éthyle comme éluant, puis recristallisé dans un mélange acétate d'éthyle-éther isopropylique (1-8 en volume). On obtient ainsi 1,7 g de méthoxy-4 [(phényl-2 quinolyl-4) oxyacétyl]-1 pipéridine fondant à 96°C.

*Exemple 21*

On opère comme à l'exemple 1 à partir de 3 g d'acide (phényl-2 quinolyl-4) oxyacétique, de 3,8 $cm^3$ de chlorure de thionyle dans 90 $cm^3$ de chloroforme, de 1,25 g de N-méthylamino-4 morpholine et de 3,25 $cm^3$ de triéthylamine dans 90 $cm^3$ de chloroforme.

Après recristallisation du résidu dans l'acétonitrile, on isole 2 g de N-méthyl N-morpholinyl-4 (phényl-2 quinolyl-4) oxyacétamide fondant à 189°C.

La N-méthylamino-4 morpholine peut être préparée selon O. ZINNGR, H. BOEHLKE et W. KLIEGEL Arch. Pharm. 299, 245-53 (1966).

*Exemple 22*

On opère comme à l'exemple 1 à partir de 3 g d'acide (phényl-2 quinolyl-4) oxyacétique, de 3,8 $cm^3$ de chlorure de thionyle dans 90 $cm^3$ de chloroforme, de 1,3 g de chlorhydrate de cyclopropylméthyl méthylamine et de 4,4 $cm^3$ de triéthylamine dans 90 $cm^3$ de chloroforme.

Le résidu est chromatographié sur du gel de silice au moyen d'un mélange cyclohexane-acétate d'éthyle (50-50 en volume) comme éluant.

Le résidu est repris dans l'acétone et après addition d'une solution d'acide chlorhydrique dans l'éther éthylique, on isole 1,6 g de chlorhydrate de N-cyclopropylméthyl N-méthyl (phényl-2 quinolyl-4) oxyacétamide fondant à 178°C.

*Exemple 23*

On opère comme à l'exemple 5 à partir de 6 g de (méthyl-4 phényl)-2 hydroxy-4 quinoléine dans 200 $cm^3$ de méthyléthylacétone, de 7,05 g de carbonate de potassium anhydre et de 5,84 g de (bromo-2 acétyl)-4 morpholine dans 40 $cm^3$ de méthyléthylacétone.

Après recristallisation du résidu dans l'acétate d'éthyle, on obtient 4,65 g de ([(méthyl-4 phényl)-2 quinolyl-4] oxyacétyl)-4 morpholine fondant à 146°C.

La (méthyl-4 phényl)-2 hydroxy-4 quinoléine peut être préparée par action à 140°C du méthyl-4 benzoylacétate d'éthyle (0,294 mole) sur l'aniline (0,294 mole) en présence d'acide polyphosphorique (168 g). Elle présente un point de fusion supérieur à 268°C.

*Exemple 24*

On opère comme à l'exemple 5 à partir de 2,3 g de (trifluorométhyl-3 phényl)-2 hydroxy-4 quinoléine dans 75 $cm^3$ de méthyléthylacétone, de 2,2 g de carbonate de potassium et de 1,85 g de (bromo-2 acétyl)-4 morpholine dans 15 $cm^3$ de méthyléthylacétone.

Après deux chromatographies successives du résidu sur du gel de silice, la première au moyen de l'acétate d'éthyle, la seconde en utilisant comme éluant un mélange cyclohexane-acétate d'éthyle (30-50 en volume), on isole 1,05 g de [(trifluorométhyl-3 phényl)-2 quinolyl-4 oxyacétyl]-4 morpholine fondant à 135°C.

La (trifluorométhyl-3 phényl)-2 hydroxy-4 quinoléine, peut être préparée par action à 160°C du trifluorométhyl-3 benzoyl acétate d'éthyle (0,245 mole) sur l'aniline (0,245 mole) en présence d'acide polyphosphorique (156 g). Ce produit est utilisé tel quel dans l'étape suivante.

*Exemple 25*

On opère comme à l'exemple 5 à partir de 4,1 g de (fluoro-2 phényl)-2 hydroxy-4 quinoléine dans 130 $cm^3$ de méthyléthylacétone, de 4,8 g de carbonate de potassium anhydre et de 3,95 g de (bromo-2 acétyl)-4 morpholine dans 25 $cm^3$ de méthyléthylacétone.

Après cristallisation du résidu dans l'éther de pétrole 40-60°, puis recristallisation dans l'acétate d'éthyle, on isole 2,5 g de ([(-fluoro-2 phényl)-2 quinolyl-4] oxyacétyl)-4 morpholine fondant à 145°C.

La (fluoro-2 phényl)-2 hydroxy-4 quinoléine peut être préparée par action, à 160°C du fluoro-2 benzoylacétate d'éthyle (0,05 mole) sur l'aniline (0,05 mole) en présence d'acide polyphosphorique (25 g).

Elle présente un point de fusion égal à 224°C.

*Exemple 26*

On opère comme à l'exemple 5 à partir de 3,4 g de (thiényl-2)-2 hydroxy-4 quinoléine dans 110 $cm^3$ de méthyléthylacétone, de 4,15 g de carbonate de potassium anhydre et de 3,45 g de (bromo-2 acétyl)-4 morpholine dans 20 $cm^3$ de méthyléthylacétone.

Le résidu est repris dans l'éther éthylique et après addition d'une solution d'acide chlorhydrique dans l'isopropanol, on isole un chlorhydrate brut. Ce dernier est repris par 100 $cm^3$ d'eau, 100 $cm^3$ de chlorure de méthylène et 30 $cm^3$ d'une solution normale d'hydroxyde de sodium. La phase organique est décantée, lavée à l'eau, séchée sur sulfate de magnésium et évaporée sous pression réduite. Le résidu est agité 15 minutes dans l'éther éthylique. Après filtration et séchage, on isole 2,15 g de ([(thiényl-2)-2 quinolyl-4] oxyacétyl)-4 morpholine fondant à 198°C.

La (thiényl-2)-2 hydroxy-4 quinoléine peut être préparée par action à 160°C du thénoyl-2 acétate d'éthyle (0,103 mole) sur l'aniline (0,103 mole) en présence d'acide polyphosphorique (45,8 g).

Elle présente un point de fusion supérieur à 268°C.

*Exemple 27*

On opère comme à l'exemple 5 à partir de 1,9 g de (chloro-3 phényl)-2 hydroxy-4 quinoléine dans 50 $cm^3$ de méthyléthylacétone, de 2,05 g de carbonate de potassium et de 1,7 g de (bromo-2 acétyl)-4 morpholine dans 10 $cm^3$ de méthyléthylacétone.

Après chromatographie du résidu sur du gel de silice au moyen d'un mélange cyclohexane-acétate d'éthyle (50-50 en volume) comme éluant et cristallisation dans l'éther de pétrole 40-60°, on isole 1,4 g de ([(chloro-3 phényl)-2 quinolyl-4] oxyacétyl)-4 morpholine fondant à 131°C.

La (chloro-3 phényl)-2 hydroxy-4 quinoléine peut être préparée par action à 160°C du chloro-3 benzoylacétate d'éthyle (0,025 mole) sur l'aniline (0,025 mole) en présence d'acide polyphosphorique (11 g).

Elle présente un point de fusion égal à 210°C.

*Exemple 28*

On opère comme à l'exemple 5 à partir de 2,22 g de (pyridyl-2)-2 hydroxy-4 quinoléine dans 70 $cm^3$ de méthyléthylacétone, de 2,76 g de carbonate de potassium anhydre et de 2,3 g de (bromo-2 acétyl)-4 morpholine dans 15 $cm^3$ de méthyléthylacétone.

Après chromatographie du résidu sur du gel de silice en utilisant un mélange méthanol-acétate d'éthyle (50-50 en volume) comme éluant, le solide obtenu est recristallisé dans l'acétate d'éthyle. On isole ainsi 1,8 g de ([(pyridyl-2)-2 quinolyl-4] oxyacétyl)-4 morpholine formule 185°C.

La (pyridyl-2)-2 hydroxy-4 quinoléine peut être préparée par action, à 160°C, du pyridinoyl-2 acétate d'éthyle (0,05 mole) sur l'aniline (0,05 mole) en présence d'acide polyphosphorique (58 g).

Elle présente un point de fusion égal à 228°C.

*Exemple 29*

A une suspension de 6 g de [(phényl-2 quinolyl-4) oxyacétyl]-4 pipérazinone-2 préparée selon l'exemple 19 dans 120 $cm^3$ de toluène et 50 $cm^3$ de diméthylformamide, on ajoute 20 $cm^3$ de diméthylsulfoxyde puis 0,73 g d'hydrure de sodium en dispersion à 60% dans l'huile.

On agite 3 heures 30 minutes à température ambiante (20°C environ), ajoute 1,13 $cm^3$ d'iodure de méthyle et agite ensuite 40 heures à température ambiante. On ajoute ensuite 200 $cm^3$ d'eau et extrait par 3 fois 150 $cm^3$ d'acétate d'éthyle. La phase organique est lavée à l'eau, séchée sur sulfate

de magnésium et évaporée sous pression réduite. Le résidu est chromatographié sur du gel de silice en utilisant un mélange acétate d'éthyle-éthanol (95-5 en volume) comme éluant. On isole ainsi 3,5 g de méthyl-1 [(phényl-2 quinolyl-4) oxyacétyl]-4 pipérazinone-2 fondant à 140°C.

*Exemple 30*

On opère comme à l'exemple 1 à partir de 6 g d'acide (phényl-2 quinolyl-4) oxyacétique, de 4,6 $cm^3$ de chlorure de thionyle dans 180 $cm^3$ de chloroforme, de 2,4 g de formyl-1 pipérazine et de 6,5 $cm^3$ de triéthylamine dans 200 $cm^3$ de chloroforme.

Après deux recristallisations la première dans l'acétate d'éthyle, la seconde dans l'éthanol, on isole 2,6 g de formyl-1 [(phényl-2 quinolyl-4) oxyacétyl]-4 pipérazine fondant à 170°C.

*Exemple 31*

On opère comme à l'exemple 5 à partir de 5,32 g de (nitro-4 phényl)-2 hydroxy-4 quinoléine dans 165 $cm^3$ de méthyléthylacétone, de 5,5 g de carbonate de potassium et de 4,6 g de (bromo-2 acétyl)-4 morpholine dans 32 $cm^3$ de méthyléthylacétone.

Après chromatographie du résidu sur du gel de silice en utilisant comme éluant un mélange cyclohexane-acétate d'éthyle (30-70 en volume), on isole un solide que l'on agite dans l'éther de pétrole 40-60°. On isole 4,8 g de ([(nitro-4 phényl)-2 quinolyl-4] oxyacétyl)-4 morpholine fondant à 172°C.

La (nitro-4 phényl)-2 hydroxy-4 quinoléine peut être préparée selon ELDERFIELD et al., J. Amer. Chem. Soc., 68, 1272 (1946).

*Exemple 32*

On opère comme à l'exemple 5 à partir de 6 g d'un mélange 50-50 en poids de chloro-5 phényl-2 quinolinol-4 et de chloro-7 phényl-2 quinolino-4, de 4,88 g de (bromo-2 acétyl)-4 morpholine et de 6,3 g de carbonate de potassium dans 200 $cm^3$ de méthyléthylacétone.

Après chromatographie du résidu sur du gel de silice au moyen d'un mélange chloroforme-acétate d'éthyle (50-50 en volume), on isole un résidu que l'on recristallise dans l'acétate d'éthyle. On obtient ainsi 1,8 g de [(chloro-5 phényl-2 quinolyl-4) oxyacétyl]-4 morpholine fondant à 168°C.

Le mélange de chloro-5 phényl-2 quinolinol-4 et de chloro-7 phényl-2 quinolinol-4 peut être obtenu par action de la chloro-3 aniline (13 g) sur le benzoylacétate d'éthyle (19,8 g) en présence d'acide polyphosphorique (70 g).

*Exemple 33*

On opère comme à l'exemple 5 à partir de 3 g de chloro-6 phényl-2 quinolinol-4, de 2,5 g de (bromo-2 acétyl)-4 morpholine et de 3,2 g de carbonate de potassium dans 100 $cm^3$ de méthyléthylacétone.

Après recristallisation du résidu dans l'acétate d'éthyle, on isole 2,9 g de [(chloro-6 phényl-2 quinolyl-4) oxyacétyl]-4 morpholine fondant à 182°C.

Le chloro-6 phényl-2 quinolinol-4 peut être préparé selon B. STASKUN et al., J. Org. Chem. 1961, (26), 3191.

*Exemple 34*

On opère comme à l'exemple 1 à partir de 6 g d'acide (phényl-2 quinolyl-4) oxyacétique, de 4,57 $cm^3$ de chlorure de thionyle dans 150 $cm^3$ de chloroforme, de 1,53 g de pyrrolidine et de 6 $cm^3$ de triéthylamine dans 150 $cm^3$ de chloroforme et en réduisant à 3 heures le temps de préparation du chlorure d'acide.

Après recristallisation du résidu dans l'acétate d'éthyle, on isole 5,3 g de [(phényl-2 quinolyl-4) oxyacétyl]-1 pyrrolidine fondant à 135°C.

*Exemple 35*

On opère comme à l'exemple 1 à partir de 6 g d'acide (phényl-2 quinolyl-4) oxyacétique, de 4,57 $cm^3$ de chlorure de thionyle dans 150 $cm^3$ de chloroforme, de 2,3 g de N-méthylaniline et de 6 $cm^3$ de triéthylamine dans 100 $cm^3$ de chloroforme et en réduisant à 3 heures le temps de préparation du chlorure d'acide.

Après recristallisation du résidu dans l'acétate d'éthyle, on obtient 5,4 g de N-méthyl N-phényl (phényl-2 quinolyl-4) oxyacétamide fondant à 150°C.

*Exemple 36*

On opère comme à l'exemple 5 à partir de 3 g de méthyl-6 phényl-2 hydroxy-4 quinoléine, de 2,4 g de (bromo-2 acétyl)-4 morpholine et de 3,5 g de carbonate de potassium dans 100 $cm^3$ de méthyléthylacétone.

Après recristallisation du résidu dans l'acétate d'éthyle, on obtient 2,9 g de [(méthyl-6 phényl-2 quinolyl-4) oxyacétyl]-4 morpholine fondant à 158°C.

Le méthyl-6 phényl-2 quinolinol-4 peut être préparé selon B. STASKUN et al., J. Org. Chem. 1961, (26), 3191.

*Exemple 37*

On opère comme à l'exemple 1 à partir de 6 g d'acide (phényl-2 quinolyl-4) oxyacétique, de 4,57 $cm^3$ de chlorure de thionyle dans 180 $cm^3$ de chloroforme, de 2 g de méthyl-3 pipéridine et de 6,48 g de triéthylamine dans 200 $cm^3$ de chloroforme et en réduisant à 3 heures le temps de préparation de chlorure d'acide.

Après chromatographie du résidu sur du gel de silice en utilisant comme éluant l'acétate d'éthyle, on isole 4,25 g de méthyl-3 (phényl-2 quinolyl-4) oxyacétyl]-1 pipéridine fondant à 96°C.

*Exemple 38*

On opère comme à l'exemple 1 à partir de 6 g d'acide (phényl-2 quinolyl-4) oxyacétique, de 4,57 $cm^3$ de chlorure de thionyle dans 180 $cm^3$ de chloroforme, de 3,21 g d'isonipécotate d'éthyle et de 6,5 $cm^3$ de triéthylamine dans 200 $cm^3$ de chloroforme et en réduisant à 3 heures le temps de préparation du chlorure d'acide.

Après chromatographie du résidu sur du gel de silice en utilisant l'acétate d'éthyle comme éluant on isole 5,5 g de [(phényl-2 quinolyl-4) oxyacétyl]-1 pipéridine carboxylate-4 d'éthyle fondant à 120°C.

*Exemple 39*

On chauffe au reflux 10 minutes, 12,5 g de [(nitro-6 phényl-2 quinolyl-4) oxyacétyl]-4 morpholine dans 550 $cm^3$ d'éthanol et on ajoute en 25 minutes une solution de 27,7 g de dithionite de sodium dans 277 $cm^3$ d'eau. On refroidit à température ambiante (20°C environ) et élimine l'éthanol sous pression réduite. La phase aqueuse est agitée avec de l'acétate d'éthyle et la phase organique est lavée à l'eau, séchée sur sulfate de magnésium et évaporée sous pression réduite. Le résidu obtenu est repris dans l'éther éthylique et transformé en chlorhydrate brut par addition d'une solution d'acide chlorhydrique dans l'isopropanol. Ce dernier est repris par l'acétate d'éthyle et une solution aqueuse de carbonate de sodium. Le précipité qui se sépare est filtré, chauffé 25 minutes dans l'acétate d'éthyle au reflux, filtré et séché. On isole ainsi 1,15 g d'[(amino-6 phényl-2 quinolyl-4) oxyacétyl]-4 morpholine fondant à 194°C.

La [(nitro-6 phényl-2 quinolyl-4) oxyacétyl]-4 morpholine peut être préparée par le procédé décrit à l'exemple 5, à partir de nitro-6 phényl-2 hydroxy-4 quinoléine (0,133 mole), de carbonate de potassium (0,265 mole) et de bromoacétyl-4 morpholine (0,146 mole) dans la méthyléthylacétone. Elle présente un point de fusion de 250°C.

*Exemple 40*

A une solution agitée de 2,55 g d'[(amino-6 phényl-2 quinolyl-4) oxyacétyl]-4 morpholine préparé selon l'exemple 39 et 1,2 $cm^3$ de triéthylamine dans 25 $cm^3$ de chlorure de méthylène, on ajoute en 10 minutes une solution de 0,55 $cm^3$ de chlorure d'acétyle dans 5 $cm^3$ de chlorure de méthylène. On agite 1 heure à température ambiante (20°C environ) et évapore le solvant sous pression réduite. Le résidu est agité 10 minutes dans un mélange acétate d'éthyle-eau (5-2 en volume), filtré, dissous dans un mélange toluène-acide acétique (50-50 en volume) et précipité par de l'éther éthylique. Après filtration et séchage, on isole 2 g d'[(acétylamino-6 phényl-2 quinolyl-4) oxyacétyl]-4 morpholine fondant à 261°C.

*Exemple 41*

On hydrogène à 40°C pendant 30 minutes puis à température ambiante (20°C environ) et sous pression atmosphérique 3,93 g de ([(nitro-4 phényl)-2 quinolyl-4] oxyacétyl)-4 morpholine préparé selon l'exemple 31 dissous dans 80 $cm^3$ de méthanol en présence de 4 $cm^3$ d'une solution 5N d'acide chlorhydrique dans l'isopropanol et de 0,4 g de charbon palladié à 10% comme catalyseur. Après absorption de la quantité théorique d'hydrogène, on ajoute au mélange réactionnel 25 $cm^3$ d'une solution normale d'hydroxyde de sodium afin de redissoudre le précipité formé. Après filtration, le solvant est éliminé sous pression réduite et la phase aqueuse résiduelle est reprise par du chlorure de méthylène. La phase organique est lavée à l'eau, séchée sur sulfate de magnésium et évaporée sous pression réduite. Le résidu (2,9 g) est dissous dans 15 $cm^3$ d'acétate d'éthyle à chaud; on ajoute ensuite 30 $cm^3$ d'éther éthylique, filtre le précipité que l'on chromatographie sur du gel de silice en utilisant un mélange toluène-méthanol-diéthylamine (90-5-5 en volume). On isole ainsi 1,05 g d'([(amino-4 phényl)-2 quinolyl-4] oxyacétyl)-4 morpholine fondant à 174°C.

*Exemple 42*

On opère comme à l'exemple 5 à partir de 2,85 g de (thiényl-3)-2 hydroxy-4 quinoléine dans 90 $cm^3$ de méthyléthylacétone, de 3,5 g de carbonate de potassium et de 2,9 g de (bromo-2 acétyl)-4 morpholine dans 18 $cm^3$ de méthyléthylacétone.

Après deux recristallisations du résidu la première dans l'acétate d'éthyle, la seconde dans l'éthanol, on isole 1,7 g de ([(thiényl-3)-2 quinolyl-4] oxyacétyl)-4 morpholine fondant à 183°C.

La (thiényl-3)-2 hydroxy-4 quinoléine peut être préparée par la méthode décrite par BOGDANOWCZ et al., Roczniki Chemii, 48, 1255 (1974), à partir de théonyl-3 acétate d'éthyle, d'aniline et d'acide polyphosphorique. Elle présente un point de fusion supérieur à 264°C.

*Exemple 43*

On opère comme à l'exemple 5 à partir de 1,6 g de phényl-2 hydroxy-4 quinoléine dans 50 $cm^3$ de méthyléthylacétone, de 2 g de carbonate de potassium et de 1,65 g de N-méthyl N-tertbutyl bromo-2 acétamide dans 10 $cm^3$ de méthyléthylacétone.

Le résidu est transformé en un chlorhydrate brut par addition d'une solution d'acide chlorhydrique dans l'isopropanol. La base est régénérée par addition d'une solution de carbonate de potassium et extraite par l'acétate d'éthyle. La phase organique est lavée à l'eau, séchée sur sulfate de magnésium et évaporée sous pression réduite. Le résidu est cristallisé dans l'éther de pétrole. On isole ainsi 1,65 g de N-méthyl N-tertbutyl (phényl-2 quinolyl-4) oxyacétamide fondant à 90°C.

La N-méthyl N-tertbutyl bromo-2 acétamide peut être préparée par action du chlorure de bromoacétyle (0,02 mole) sur la N-tertbutylméthylamine (0,02 mole) en présence de triéthylamine (0,022 mole) au sein du chlorure de méthylène.

*Exemple 44*

On opère comme à l'exemple 1 à partir de 19,7 g d'acide (phényl-2 quinolyl-4) oxyacétique, de 15,45 $cm^3$ de chlorure de thionyle dans 100 $cm^3$ de toluène, de 6 g de N-méthyl cyclobutylamine et de 60 $cm^3$ de triéthylamine dans 200 $cm^3$ de toluène et en réduisant à 2 heures le temps de préparation du chlorure d'acide.

Après chromatographie du résidu sur du gel de silice en utilisant comme éluant un mélange cyclohexane-acétate d'éthyle (70-30 en volume), on obtient un résidu que l'on reprend dans l'éther de pétrole 40-60°. Après filtration et séchage, on obtient 12,65 g de N-cyclobutyl N-méthyl (phényl-2 quinolyl-4) oxyacétamide fondant à 110°C.

La N-méthylcyclobutylamine peut être préparée par adaptation de la méthode décrite par S.F. BLICKE et al., J. Amer. Chem. Soc. (74), 3933-34 (1952).

Les médicaments selon l'invention sont constitués par un composé de formule (I), un mélange de com-

posés stéréoisomères de formule (I), ou lorsqu'il peut exister un sel d'un tel composé ou mélange, à l'état pur ou sous forme d'une composition dans laquelle il est associé à tout produit pharmaceutiquement compatible, pouvant être inerte ou physiologiquement actif. Ces médicaments selon l'invention peuvent être usilisés par voie orale, parentérale, rectale ou topique.

Comme compositions solides pour administration orale peuvent être utilisés des comprimés, pilules, poudres (capsules de gélatine, cachets) ou granulés. Dans ces compositions, le principe actif selon l'invention est mélangé à un ou plusieurs diluants inertes, tels que amidon, cellulose, saccharose, lactose ou silice. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale peuvent être de préférence des solutions non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peur se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales, qui contiennent outre le produit actif des excipients tels que le beurre de cacao, des glycérides semi-synthétiques ou des polyéthylèneglycols.

Les compositions pour administration topique peuvent être par exemple des crèmes, pommades, lotions, collyres, collutoires, gouttes nasales ou aérosols.

En thérapeutiques humaine, les composés selon l'invention sont particulièrement utiles comme anxiolytiques.

Les doses dépendent de l'effet recherché, de la durée du traitement et de la voie d'administration utilisée; elles sont généralement comprises entre 20 et 1000 mg par jour par voie orale pour un adulte avec des doses unitaires de 5 à 200 mg de substance active.

D'une façon générale, le médicin déterminera la posologie appropriée en fonction de l'âge, du poids et de tous les autres facteurs propres au sujet à traiter.

Les exemples suivants illustrent des compositions selon l'invention:

*Exemple A*

On prépare, selon la technique habituelle, des gélules dosées à 59 mg de produit actif ayant la composition suivante:

| | |
|---|---|
| - [(phényl-2 quinolyl-4) oxy acétyl]-4 morpholine | 50 mg |
| - Cellulose | 18 mg |
| - Lactose | 55 mg |
| - Silice colloïdale | 1 mg |
| - Carboxyméthylamidon sodique | 10 mg |
| - Talc | 10 mg |
| - Stéarate de magnésium | 1 mg |

*Exemple B*

On prépare, selon la technique habituelle, des comprimés dosés à 50 mg de produit actif ayant la composition sivante:

| | |
|---|---|
| - N-benzyl N-méthyl (phényl-2 quinolyl-4) oxyacétamide | 50 mg |
| - Lactose | 104 mg |
| - Cellulose | 40 mg |
| - Polyvidone | 10 mg |
| - Carboxyméthylamidon sodique | 22 mg |
| - Talc | 10 mg |
| - Stéarate de magnésium | 2 mg |
| - Silice colloïdale | 2 mg |
| - Mélange d'hydroxyméthylcellulose, glycérine, oxyde de titane (72-3, 5-24,5) | q.s.q 1 comprimé pelliculé terminé à 245 mg. |

*Exemple C*

On prépare une solution injectable contenant 10 mg de produit actif ayant la composition suivante:

| | | |
|---|---|---|
| - [(phényl-2 quinolyl-4) oxy acétyl]-4 morpholine | | 10 mg |
| - Acide benzoïque | | 80 mg |
| - Alcool benzylique | | 0,06 $cm^3$ |
| - Benzoate de sodium | | 80 mg |
| - Ethanol | | 0,4 $cm^3$ |
| - Hydroxyde de sodium | | 24 mg |
| - Propylène glycol | | 1,6 $cm^3$ |
| - Eau | q.s.q | 4 $cm^3$ |

**Revendications pour les Etats contractants:**
BE, CH, DE, FR, GB, IT, LI, LU, NL, SE

1. Composé racémique ou stéréoisomère de formule (I)

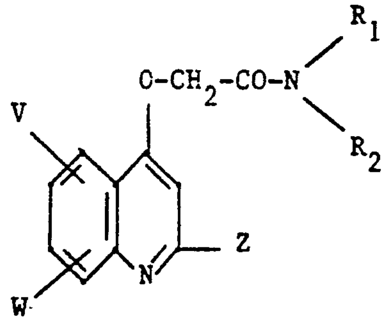

(I)

dans laquelle V et W, identiques ou différents, représentent des atomes d'hydrogène ou d'halogène, des groupes alkyle comportant 1 à 4 atomes de carbone, alcoxy comportant 1 à 4 atomes de carbone, amino ou acylamino comportant 1 à 4 atomes de carbone,

Z représente un radical phényle, thiényle, pyridyle ou phényle substitué par un ou deux substituants pris parmi les atomes d'halogène, les groupes alkyle et alcoxy comportant 1 à 4 atomes de carbone, les groupes trifluorométhyle, nitro ou amino,

$R_1$ représente un groupe alkyle linéaire ou ramifié comportant 1 à 6 atomes de carbone, alcoxyalkyle dont les parties alkyle et alcoxy comportent chacune 1 à 4 atomes de carbone, cycloalkyle comportant 3 à 6 atomes de carbone, phényle, phénylalkyle dont la partie alkyle comporte 1 à 4 atomes de carbone ou cycloalkylalkyle dont la partie alkyle comporte 1 à 3 atomes de carbone et la partie cycloalkyle comporte 3 à 6 atomes de carbone,

$R_2$ représente un groupe alkyle linéaire ou ramifié comportant 1 à 6 atomes de carbone, alcoxyalkyle dont les parties alcoxy et alkyle comportent chacune 1 à 4 atomes de carbone, cycloalkyle comportant 3 à 6 atomes de carbone, phényle, phénylalkyle dont la partie alkyle comporte 1 à 4 atomes de carbone ou cycloalkylalkyle dont la partie alkyle comporte 1 à 3 atomes de carbone et la partie cycloalkyle comporte 3 à 6 atomes de carbone ou un cycle morpholine-4,

$R_1$ et $R_2$ peuvent également former ensemble avec l'atome d'azote auquel ils sont rattachés un cycle pyrrolidine, pipéridine éventuellement substitué par un groupe hydroxy, alkyle comportant 1 à 4 atomes de carbone, alcoxy comportant 1 à 4 atomes de carbone, alkyloxycarbonyle dont la partie alkyle comporte 1 à 4 atomes de carbone, un cycle morpholine éventuellement substitué par un ou deux groupes alkyle comportant 1 à 4 atomes de carbone, un cycle thiomorpholine, un cycle pipérazine éventuellement substitué sur l'atome d'azote par un groupe alkyle comportant 1 à 4 atomes de carbone, alkyloxycarbonyle dont la partie alkyle comporte 1 à 4 atomes de carbone, acyle comportant 2 à 5 atomes de carbone, formyle ou un cycle pipérazinone éventuellement substitué sur l'atome d'azote par un groupe alkyle comportant 1 à 4 atomes de carbone, ainsi que lorsqu'il peut exister un sel d'un composé avec un acide.

2. Procédé de préparation d'un composé selon la revendication 1 caractérisé en ce que l'on fait réagir une amine de formule:

$$HN{<}^{R_1}_{R_2} \qquad (II)$$

dans laquelle $R_1$ et $R_2$ ont les mêmes significations que dans la formule (I) ou un sel de cette amine, sur un dérivé de formule:

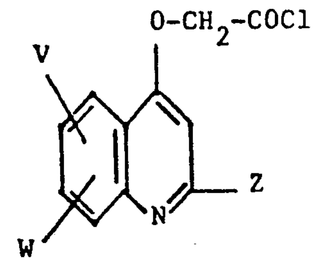

(III)

dans laquelle V,W et Z ont les mêmes significations que dans la formule (I), isole le produit et le transforme éventuellement en un sel d'addition avec un acide.

3. Procédé de préparation d'un composé selon la revendication 1 dans la formule duquel V et W, identiques ou différents, représentent des atomes d'hydrogène ou d'halogène, des groupes alkyle comportant 1 à 4 atomes de carbone ou alcoxy comportant 1 à 4 atomes de carbone, Z représente un radical phényle, thiényle, pyridyle ou phényle substitué par un ou deux substituants pris parmi les atomes d'halogène, les groupes alkyle et alcoxy comportant 1 à 4 atomes de carbone, les groupes trifluorométhyle ou nitro, $R_1$ et $R_2$ sont définis comme à la revendication 1 caractérisé en ce que l'on fait réagir un composé de formule:

$$Hal\text{-}CH_2\text{-}CO\text{-}N{<}^{R_1}_{R_2} \qquad (V)$$

dans laquelle $R_1$ et $R_2$ sont définis comme dans la formule (I) et Hal représente un atome d'halogène, sur un dérivé de formule:

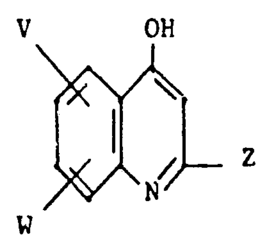

(VI)

dans laquelle V, W et Z sont définis comme précédemment, isole le produit et le transforme éventuellement en sel d'addition avec un acide.

4. Procédé de préparation d'un composé selon la revendication 1 dans la formule duquel V et W, identiques ou différents, représentent des atomes d'hydrogène ou d'halogène, des groupes alkyle comportant 1 à 4 atomes de carbone ou alcoxy comportant 1 à 4 atomes de carbone, Z représente un radical phényle, thiényle, pyridyle ou phényle substitué par un ou deux substituants pris parmis les atomes d'halogène, les groupes alkyle et alcoxy comportant 1 à 4 atomes de carbone, les groupes trifluorométhyle ou nitro et $NR_1R_2$ représente un cycle pipéridine substitué par un groupe alcoxy comportant 1 à 4 atomes de carbone caractérisé en ce que l'on fait réagir un composé de formule:

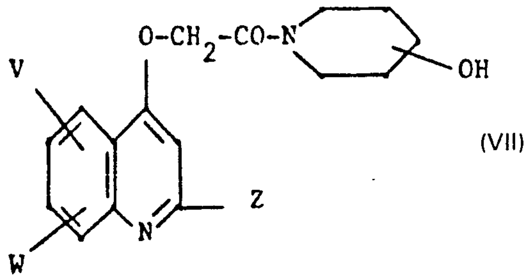

(VII)

sur un dérivé de formule:

Hal-R (VIII)

dans lesquelles V, W et Z sont définis comme précédemment, Hal représente un atome d'halogène et R représente un groupe alkyle comportant 1 à 4 atomes de carbone, isole le produit et le transforme éventuellement en sel d'addition avec un acide.

5. Procédé de préparation d'un composé selon la revendication 1 dans la formule duquel V et W, identiques ou différents, représentent des atomes d'hydrogène ou d'halogène, des groupes alkyle comportant 1 à 4 atomes de carbone ou alcoxy comportant 1 à 4 atomes de carbone, Z représente un radical phényle, thiényle, pyridyle ou phényle substitué par un ou deux substituants pris parmi les atomes d'halogène, les groupes alkyle et alcoxy comportant 1 à 4 atomes de carbone, les groupes trifluorométhyle ou nitro et $NR_1R_2$ représente un cycle pipérazinone substitué sur l'atome d'azote par un groupe alkyle comportant 1 à 4 atomes de carbone caractérisé en ce que l'on fait réagir un composé correspondant de formule (I) dans laquelle $NR_1R_2$ représente un cycle pipérazinone avec un halogénure d'alkyle de formule Hal-R (VIII) dans laquelle Hal représente un atome d'halogène et R représente un groupe alkyle comportant 1 à 4 atomes de carbone, isole le produit et le transforme éventuellement en sel d'addition avec un acide.

6. Procédé de préparation d'un composé selon la revendication 1 dans la formule duquel V et/ou W sont des groupes amino et/ou Z est un radical phényle substitué par un ou deux groupes amino, $R_1$ et $R_2$ sont définis comme dans la revendication 1, étant entendu que ces composés sont exempts de groupe nitro, caractérisé en ce que l'on hydrogène catalytiquement le dérivé nitré correspondant, isole le produit et le transforme éventuellement en sel d'addition avec un acide.

7. Procédé de préparation d'un composé selon la revendication 1 dans la formule duquel V et/ou W sont des groupes amino et/ou Z est un radical phényle substitué par un ou deux groupes amino, $R_1$ et $R_2$ sont définis comme dans la revendication 1 étant entendu que ces composés sont exempts de groupe nitro, caractérisé en ce que l'on réduit le dérivé nitré correspondant, isole le produit et le transforme éventuellement en sel d'addition avec un acide.

8. Procédé de préparation d'un composé selon la revendication 1 dans la formule duquel V et/ou W sont des groupes alcylamino comportant 1 à 4 atomes de carbone, Z, $R_1$ et $R_2$ sont définis comme dans la revendication 1 étant entendu que Z n'est pas un radical phényle substitué par un groupe amino caractérisé en ce que l'on fait réagir le composé de formule (I) correspondant pour lequel V et/ou W sont des groupes amino avec un agent d'acylation de formule:

Hal-CO-E (IX)

dans laquelle Hal représente un atome d'halogène et E représente un atome d'hydrogène ou un groupe alkyle ayant 1 à 3 atomes de carbone, isole le produit et le transforme éventuellement en sel d'addition avec un acide.

9. Médicament contenant au moins une substance active et éventuellement un ou plusieurs diluants ou adjuvants compatibles et pharmaceutiquement acceptables, caractérisé en ce que la substance active est un composé selon la revendication 1.

**Revendication pour l'Etat contractant:** AT

Procédé de préparation d'un composé racémique ou stéréoisomère de formule (I)

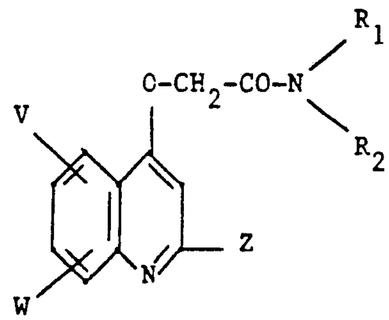

(I)

dans laquelle V et W, identiques ou différents, représentent des atomes d'hydrogène ou d'halogène, des groupes alkyle comportant 1 à 4 atomes de carbone, alcoxy comportant 1 à 4 atomes de carbone, des groupes amino ou acylamino comportant 1 à 4 atomes de carbone,

Z représente un radical phényle, thiényle, pyridyle ou phényle substitué par un ou deux substituants pris parmi les atomes d'halogène, les groupes alkyle et alcoxy comportant 1 à 4 atomes de carbone, les groupes trifluorométhyle, nitro ou amino,

$R_1$ représente un groupe alkyle linéaire ou ramifié comportant 1 à 6 atomes de carbone, alcoxyalkyle dont les parties alkyle et alcoxy comportent chacune 1 à 4 atomes de carbone, cycloalkyle comportant 3 à 6 atomes de carbone, phényle, phénylalkyle dont la partie alkyle comporte 1 à 4 atomes de carbone ou cycloalkylalkyle dont la partie alkyle comporte 1 à 3 atomes de carbone et la partie cycloalkyle comporte 3 à 6 atomes de carbone,

$R_2$ représente un groupe alkyle linéaire ou ramifié comportant 1 à 6 atomes de carbone, alcoxyalkyle dont les parties alcoxy et alkyle comportent chacune 1 à 4 atomes de carbone, cycloalkyle comportant 3 à 6 atomes de carbone, phényle, phénylalkyle dont la partie alkyle comporte 1 à 4 atomes de carbone ou cycloalkylalkyle dont la partie alkyle comporte 1 à 3

atomes de carbone et la partie cycloalkyle comporte 3 à 6 atomes de carbone ou un cycle morpholine-4,

$R_1$ et $R_2$ peuvent également former ensemble avec l'atome d'azote auquel ils sont rattachés un cycle pyrrolidine, pipéridine éventuellement substitué par un groupe hydroxy, alkyle comportant 1 à 4 atomes de carbone, alcoxy comportant 1 à 4 atomes de carbone, alkyloxycarbonyle dont la partie alkyle comporte 1 à 4 atomes de carbone, un cycle morpholine éventuellement substitué par un ou deux groupes alkyle comportant 1 à 4 atomes de carbone, un cycle thiomorpholine, un cycle pipérazine éventuellement substitué sur l'atome d'azote par un groupe alkyle comportant 1 à 4 atomes de carbone, alkyloxycarbonyle dont la partie alkyle comporte 1 à 4 atomes de carbone, acyle comportant 2 à 5 atomes de carbone, formyle ou un cycle pipérazinone éventuellement substitué sur l'atome d'azote par un groupe alkyle comportant 1 à 4 atomes de carbone, ainsi que lorsqu'il peut exister un sel d'un composé avec un acide, caractérisé en ce que:

a - pour la préparation d'un composé de formule (I) dans laquelle V, W, Z, $R_1$ et $R_2$ ont toutes les significations mentionnées précédemment, on fait réagir une amine de formule:

$$HN{<}^{R_1}_{R_2} \qquad (II)$$

dans laquelle $R_1$ et $R_2$ ont les mêmes significations que dans la formule (I), ou un sel de cette amine, sur un dérivé de formule:

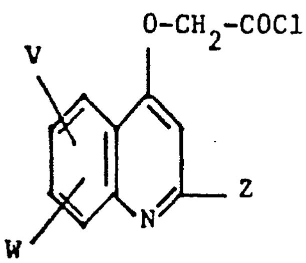

(III)

dans laquelle V, W et Z ont les mêmes significations que dans la formule (I), isole le produit et le transforme éventuellement en sel d'addition avec un acide,

b - pour la préparation d'un composé de formule (I) dans laquelle V et W, identiques ou différents, représentent des atomes d'hydrogène ou d'halogène, des groupes alkyle comportant 1 à 4 atomes de carbone ou alcoxy comportant 1 à 4 atomes de carbone, Z représente un radical phényle, thiényle, pyridyle ou phényle substitué par un ou deux substituants pris parmi les atomes d'halogène, les groupes alkyle et alcoxy comportant 1 à 4 atomes de carbone, les groupes trifluorométhyle ou nitro, $R_1$ et $R_2$ sont définis comme précédemment, on fait réagir un composé de formule:

$$Hal\text{-}CH_2\text{-}CO\text{-}N{<}^{R_1}_{R_2} \qquad (V)$$

dans laquelle $R_1$ et $R_2$ sont définis comme dans la formule (I) et Hal représente un atome d'halogène, sur un dérivé de formule:

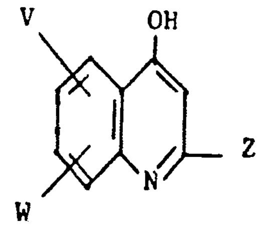

(VI)

dans laquelle V, W et Z sont définis comme précédemment, isole le produit et le transforme éventuellement en sel d'addition avec un acide,

c - pour la préparation d'un composé de formule (I) dans laquelle V et W, identiques ou différents, représentent des atomes d'hydrogène ou d'halogène, des groupes alkyle comportant 1 à 4 atomes de carbone ou alcoxy comportant 1 à 4 atomes de carbone, Z représente un radical phényle, thiényle, pyridyle ou phényle substitué par un ou deux substituants pris parmis les atomes d'halogène, les groupes alkyle et alcoxy comportant 1 à 4 atomes de carbone, les groupes trifluorométhyle ou nitro et $NR_1R_2$ représente un cycle pipéridine substitué par un groupe alcoxy comportant 1 à 4 atomes de carbone, on fait réagir un composé de formule:

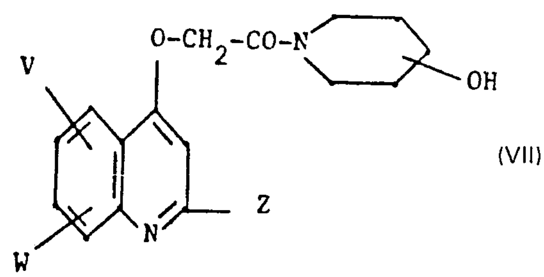

(VII)

sur un dérivé de formule:

Hal-R (VIII)

dans lesquelles V, W et Z sont définis comme précédemment, Hal représente un atome d'halogène et R représente un groupe alkyle comportant 1 à 4 atomes de carbone, isole le produit et le transforme éventuellement en sel d'addition avec un acide,

d - pour la préparation d'un composé de formule (I) dans laquelle V et W, identiques ou différents, représentent des atomes d'hydrogène ou d'halogène, des groupes alkyle comportant 1 à 4 atomes de carbone, ou alcoxy comportant 1 à 4 atomes de carbone, Z représente un radical phényle, thiényle, pyridyle ou phényle substitué par un ou deux substituants pris parmi les atomes d'halogène, les groupes alkyle et alcoxy comportant 1 à 4 atomes de carbone, les groupes trifluorométhyle ou nitro et $NR_1R_2$ représente un cycle pipérazinone substitué sur l'atome d'azote par un groupe alkyle comportant1 à 4 atomes de carbone, on fait réagir un composé correspondant de formule (I) dans laquelle $NR_1R_2$ représente un cycle pipérazinone avec un dérivé de formule:

Hal-R (VIII)

dans laquelle Hal représente un atome d'halogène et R représente un groupe alkyle comportant 1 à 4 atomes de carbone, isole le produit et le transforme éventuellement en sel d'addition avec un acide,

e - pour la préparation d'un composé de formule (I) dans laquelle V et/ou W sont des groupes amino et/ou Z est un radical phényle substitué par un ou deux groupes amino, $R_1$ et $R_2$ sont définis comme précédemment, étant entendu que ces composés sont exempts de groupe nitro, on hydrogène catalytiquement le dérivé nitré correspondant, isole le produit et le transforme éventuellement en sel d'addition avec un acide,

f - pour la préparation d'un composé de formule (I) dans laquelle V et/ou W sont des groupes amino et/ou Z est un radical phényle substitué par un ou deux groupes amino, $R_1$ et $R_2$ sont définis comme précédemment étant entendu que ces composés sont exempts de groupe nitro, on réduit le dérivé nitré correspondant, isole le produit et le transforme éventuellement en sel d'addition avec un acide,

g - pour la préparation d'un composé de formule (I) dans laquelle V et/ou W sont des groupes acylamino comportant 1 à 4 atomes de carbone, Z, $R_1$ et $R_2$ sont définis comme précédemment, étant entendu que Z n'est pas un radical phényle substitué par un groupe amino, on fait réagir le composé de formule (I) correspondant pour lequel V et/ou W sont des groupes amino avec un agent d'acylation de formule:

Hal-CO-E (IX)

dans laquelle Hal représente un atome d'halogène et E représente un atome d'hydrogène ou un groupe alkyle ayant 1 à 3 atomes de carbone, isole le produit et le transforme éventuellement en sel d'addition avec un acide.

**Patentansprüche für die Vertragsstaaten:**
BE, CH, DE, FR, GB, IT, LI, LU, NL, SE

1. Racemische oder stereoisomere Verbindung der Formel (I):

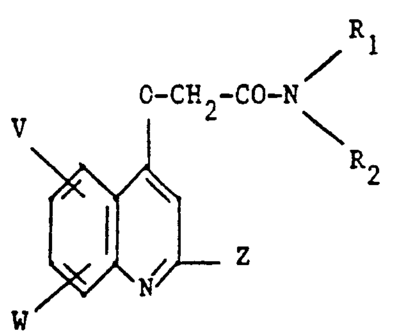

(I)

worin V und W, die identisch oder verschieden sind, Wasserstoff- oder Halogenatome, Alkylgruppen mit 1 bis 4 Kohlenstoffatomen, Alkoxygruppen mit 1 bis 4 Kohlenstoffatomen, Amino- oder Acylaminogruppen mit 1 bis 4 Kohlenstoffatomen bedeuten,

Z einen Phenyl-, Thienyl-, Pyridylrest bedeutet oder Phenyl, substituiert durch einen oder zwei Substituenten, ausgewählt unter den Halogenatomen, den Alkyl- und Alkoxygruppen mit 1 bis 4 Kohlenstoffatomen, den Trifluormethyl-, Nitro- oder Aminogruppen,

$R_1$ bedeutet eine gerade oder verzweigte Alkylgruppe mit 1 bis 6 Kohlenstoffatomen, Alkoxyalkyl, dessen Alkyl- und Alkoxyteile jeweils 1 bis 4 Kohlenstoffatome tragen, Cycloalkyl mit 3 bis 6 Kohlenstoffatomen, Phenyl, Phenylalkyl, dessen Alkylteil 1 bis 4 Kohlenstoffatome trägt, oder Cycloalkylalkyl, dessen Alkylteil 1 bis 3 Kohlenstoffatome hat und der Cycloalkylteil 3 bis 6 Kohlenstoffatome trägt,

$R_2$ bedeutet eine gerade oder verzweigte Alkylgruppe mit 1 bis 4 Kohlenstoffatomen, Alkoxyalkyl, dessen Alkoxy- und Alkylteile jeweils 1 bis 4 Kohlenstoffatome tragen, Cycloalkyl mit 3 bis 6 Kohlenstoffatomen, Phenyl, Phenylalkyl, dessen Alkylteil 1 bis 4 Kohlenstoffatome hat, oder Cycloalkylalkyl, dessen Alkylteil 1 bis 3 Kohlenstoffatome hat und der Cycloalkylteil 3 bis 6 Kohlenstoffatome hat, oder einen 4-Morpholinring,

$R_1$ und $R_2$ können auch zusammen mit dem Stickstoffatom, an das sie gebunden sind, einen Pyrrolidinring bilden, einen Piperidinring, gegebenenfalls substituiert durch eine Hydroxygruppe, Alkyl mit 1 bis 4 Kohlenstoffatomen, Alkoxy mit 1 bis 4 Kohlenstoffatomen, Alkyloxycarbonyl, dessen Alkylteil 1 bis 4 Kohlenstoffatome hat, einen Morpholinring, gegebenenfalls substituiert durch eine oder zwei Alkylgruppen mit 1 bis 4 Kohlenstoffatomen, einen Thiomorpholinring, einen Piperazinring, der gegebenenfalls am Stickstoffatom substituiert ist durch eine Alkylgruppe mit 1 bis 4 Kohlenstoffatomen, Alkyloxycarbonyl, dessen Alkylteil 1 bis 4 Kohlenstoffatome hat, Acyl mit 2 bis 5 Kohlenstoffatomen, Formyl oder einen Piperazinonring, der gegebenenfalls am Stickstoffatom durch eine Alkylgruppe mit 1 bis 4 Kohlenstoffatomen substituiert ist, sowie, falls es existieren kann, ein Salz einer solchen Verbindung mit einer Säure.

2. Verfahren zur Herstellung einer Verbindung gemäß Anspruch 1, dadurch gekennzeichnet, daß man ein Amin der Formel (II):

$$HN{<}^{R_1}_{R_2} \qquad (II)$$

worin $R_1$ und $R_2$ dieselben Bedeutungen wie in Formel (I) haben, oder ein Salz dieses Amins mit einem Derivat der Formel (III):

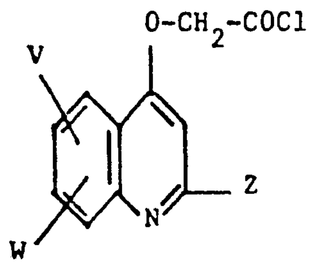

(III)

zur Reaktion bringt, worin V, W und Z dieselben Bedeutungen wie in Formel (I) haben, das Produkt isoliert und es gegebenenfalls in ein Additionssalz mit einer Säure überführt.

3. Verfahren zur Herstellung einer Verbindung gemäß Anspruch 1, in deren Formel V und W, die identisch oder verschieden sind, Wasserstoff- oder Halogenatome, Alkylgruppen mit 1 bis 4 Kohlenstoffatomen oder Alkoxygruppen mit 1 bis 4 Kohlenstoffatomen bedeuten, Z einen Phenyl-, Thienyl-, Pyridylrest bedeutet, oder Phenyl, substituiert durch einen oder zwei Substituenten, ausgewählt unter den Halogenatomen, den Alkyl- und Alkoxygruppen mit 1 bis 4 Kohlenstoffatomen, den Trifluormethyl- oder Nitrogruppen, $R_1$ und $R_2$ wie in Anspruch 1 definiert sind, dadurch gekennzeichnet, daß man eine Verbindung der Formel (V):

$$Hal-CH_2-CO-N\begin{matrix}R_1\\R_2\end{matrix} \quad (V)$$

worin $R_1$ und $R_2$ wie in Formel (I) definiert sind und Hal ein Halogenatom bedeutet, mit einem Derivat der Formel (VI):

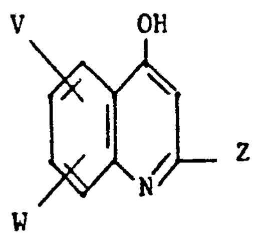

(VI)

worin V, W und Z wie vorstehend definiert sind, zur Reaktion bringt, das Produkt isoliert und es gegebenenfalls in ein Additionssalz mit einer Säure überführt.

4. Verfahren zur Herstellung einer Verbindung gemäß Anspruch 1, in deren Formel V und W, die identisch oder verschieden sind, Wasserstoff- oder Halogenatome, Alkylgruppen, mit 1 bis 4 Kohlenstoffatomen oder Alkoxygruppen mit 1 bis 4 Kohlenstoffatomen bedeuten, Z einen Phenyl-, Thienyl-, Pyridylrest bedeutet, oder Phenyl, substituiert durch einen oder zwei Substituenten, ausgewählt unter den Halogenatomen, den Alkyl- und Alkoxygruppen mit 1 bis 4 Kohlenstoffatomen, den Trifluormethyl- oder Nitrogruppen, und $NR_1R_2$ einen Piperidinring, substituiert durch eine Alkoxygruppe mit 1 bis 4 Kohlenstoffatomen, bedeutet, dadurch gekennzeichnet, daß man eine Verbindung der Formel (VII):

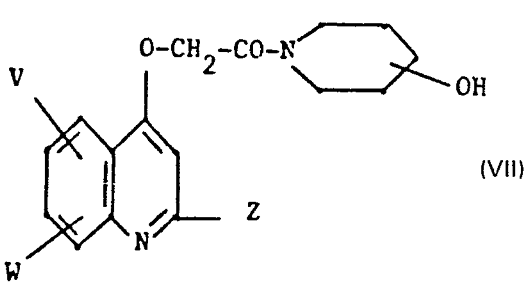

(VII)

mit einem Derivat der Formel (VIII):

$$Hal-R \quad (VIII)$$

worin V, W und Z wie vorstehend definiert sind, Hal ein Halogenatom bedeutet und R eine Alkylgruppe mit 1 bis 4 Kohlenstoffatomen darstellt, zur Reaktion bringt, das Produkt isoliert und es gegebenenfalls in ein Additionssalz mit einer Säure überführt.

5. Verfahren zur Herstellung einer Verbindung gemäß Anspruch 1, in deren Formel V und W, die identisch oder verschieden sind, Wasserstoff- oder Halogenatome, Alkylgruppen mit 1 bis 4 Kohlenstoffatomen oder Alkoxygruppen mit 1 bis 4 Kohlenstoffatomen bedeuten, Z einen Phenyl-, Thienyl-, Pyridylrest bedeutet, oder Phenyl, substituiert durch einen oder zwei Substituenten, ausgewählt unter den Halogenatomen, den Alkyl- und Alkoxygruppen mit 1 bis 4 Kohlenstoffatomen, den Trifluormethyl- oder Nitrogruppen, und $NR_1R_2$ einen Piperazinonring bedeutet, der am Stickstoffatom durch eine Alkylgruppe mit 1 bis 4 Kohlenstoffatomen substituiert ist, dadurch gekennzeichnet, daß man eine Verbindung entsprechend der Formel (I), worin $NR_1R_2$ einen Piperazinonring bedeutet, mit einem Alkylhalogenid der Formel Hal-R (VIII) umsetzt, worin Hal ein Halogenatom bedeutet und R eine Alkylgruppe mit 1 bis 4 Kohlenstoffatomen darstellt, das Produkt isoliert und es gegebenenfalls in ein Additionssalz mit einer Säure überführt.

6. Verfahren zur Herstellung einer Verbindung gemäß Anspruch 1, in deren Formel V und/oder W Aminogruppen sind, und/oder Z ein Phenylrest, substituiert durch eine oder zwei Aminogruppen, ist, $R_1$ und $R_2$ wie in Anspruch 1 definiert sind, wobei diese Verbindungen keine Nitrogruppe haben, dadurch gekennzeichnet, daß man das entsprechende nitrierte Derivat katalytisch hydriert, das Produkt isoliert und es gegebenenfalls in ein Additionssalz mit einer Säure überführt.

7. Verfahren zur Herstellung einer Verbindung gemäß Anspruch 1, in deren Formel V und/oder W Aminogruppen sind, und/oder Z ein Phenylrest, substituiert durch eine oder zwei Aminogruppen, ist, $R_1$ und $R_2$ wie in Anspruch 1 definiert sind, wobei diese Verbindungen keine Nitrogruppe aufweisen, dadurch gekennzeichnet, daß man das entsprechende nitrierte Derivat reduziert, das Produkt isoliert und es gegebenenfalls in ein Additionssalz mit einer Säure überführt.

8. Verfahren zur Herstellung einer Verbindung gemäß Anspruch 1, in deren Formel V und/oder W Acylaminogruppen mit 1 bis 4 Kohlenstoffatomen sind, Z, $R_1$ und $R_2$ wie in Anspruch 1 definiert sind, wobei Z nicht ein Phenylrest, substituiert durch eine Aminogruppe, ist, dadurch gekennzeichnet, daß man die entsprechende Verbindung der Formel (I), worin V und/oder W Aminogruppen sind, mit einem Acylierungsmittel der Formel (IX):

$$Hal-CO-E \quad (IX)$$

umsetzt, worin Hal ein Halogenatom bedeutet und E ein Wasserstoffatom oder eine Alkylgruppe mit 1 bis 3 Kohlenstoffatomen darstellt, das Produkt isoliert und es gegebenenfalls in ein Additionssalz mit einer Säure überführt.

9. Arzneimittel, enthaltend mindestens eine aktive Substanz und gegebenenfalls ein oder mehrere

verträgliche und pharmazeutisch annehmbare Verdünnungs- oder Hilfsmittel, dadurch gekennzeichnet, daß die aktive Substanz eine Verbindung gemäß Anspruch 1 ist.

**Patentanspruch für den Vertragsstaat:** AT

Verfahren zur Herstellung einer racemischen oder stereoisomeren Verbindung der Formel (I)

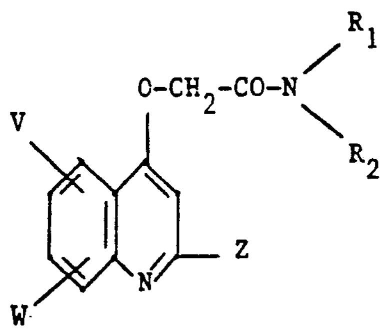

(I)

in welcher V und W unabhängig voneinander Wasserstoff- oder Halogenatome, Alkylgruppen mit 1 bis 4 Kohlenstoffatomen, Alkoxygruppen mit 1 bis 4 Kohlenstoffatomen, Aminogruppen oder Acylaminogruppen mit 1 bis 4 Kohlenstoffatomen darstellen,

Z einen Phenyl-, Thienyl-, Pyridylrest oder einen durch einen oder zwei Substituenten, ausgewählt aus den Halogenatomen, den Alkyl- oder Alkoxygruppen mit 1 bis 4 Kohlenstoffatomen, den Trifluormethyl-, Nitro- oder Aminogruppen, substituierten Phenylrest darstellt,

$R_1$ eine geradkettige oder verzweigte Alkylgruppe mit 1 bis 6 Kohlenstoffatomen, Alkoxyalkylgruppe, deren Alkyl- und Alkoxyteile jeweils 1 bis 4 Kohlenstoffatome enthalten, Cycloalkylgruppe mit 3 bis 6 Kohlenstoffatomen, Phenylgruppe, Phenylalkylgruppe, deren Alkylteil 1 bis 4 Kohlenstoffatome enthält oder Cycloalkylalkylgruppe, deren Alkylteil 1 bis 3 Kohlenstoffatome und deren Cycloalkylteil 3 bis 6 Kohlenstoffatome enthält, darstellt,

$R_2$ eine geradkettige oder verzweigte Alkylgruppe mit 1 bis 6 Kohlenstoffatomen, Alkoxyalkylgruppe, deren Alkoxy- und Alkylteile jeweils 1 bis 4 Kohlenstoffatome enthalten, Cycloalkylgruppe mit 3 bis 6 Kohlenstoffatomen, Phenylgruppe, Phenylalkylgruppe, deren Alkylteil 1 bis 4 Kohlenstoffatome enthält, Cycloalkylalkylgruppe, deren Alkylteil 1 bis 3 Kohlenstoffatome enthält und deren Cycloalkylteil 3 bis 6 Kohlenstoffatome enthält, oder einen 4-Morpholinring darstellt, wobei

$R_1$ und $R_2$ auch zusammen mit dem Stickstoffatom, an das sie gebunden sind, einen Pyrrolidinring, Piperidinring (gegebenenfalls substituiert durch eine Hydroxygruppe, Alkylgruppe mit 1 bis 4 Kohlenstoffatomen, Alkoxygruppe mit 1 bis 4 Kohlenstoffatomen, Alkoxycarbonylgruppe, deren Alkylteil 1 bis 4 Kohlenstoffatome enthält), einen Morpholinring (gegebenenfalls substituiert durch eine oder zwei Alkylgruppen mit 1 bis 4 Kohlenstoffatomen), einen Thiomorpholinring, einen Piperazinring (gegebenenfalls substituiert am Stickstoffatom durch eine Alkylgruppe mit 1 bis 4 Kohlenstoffatomen, Alkyloxycarbonylgruppe, deren Alkylteil 1 bis 4 Kohlenstoffatome enthält, Acylgruppe mit 2 bis 5 Kohlenstoffatomen, Formylgruppe) oder einen Piperazinonring (gegebenenfalls substituiert am Stickstoffatom durch eine Alkylgruppe mit 1 bis 4 Kohlenstoffatomen) bilden können, sowie, falls existent, eines Salzes einer solchen Verbindung mit einer Säure, dadurch gekennzeichnet, daß man:

a - zur Herstellung einer Verbindung der Formel (I), worin V, W, Z, $R_1$ und $R_2$ alle obigen Bedeutungen haben, ein Amin der Formel:

$$HN{<}^{R_1}_{R_2} \qquad (II)$$

in welcher $R_1$ und $R_2$ die gleichen Bedeutungen wie in der Formel (I) haben, oder ein Salz dieses Amins mit einer Verbindung der Formel:

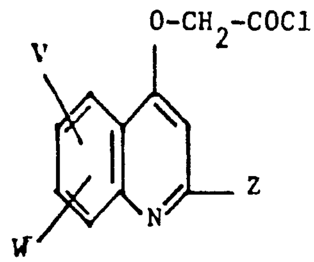

(III)

in welcher V, W und Z die gleichen Bedeutungen wie in der Formel (I) haben, reagieren läßt, das Produkt isoliert und es gegebenenfalls in ein Säureadditionssalz überführt,

b - zur Herstellung einer Verbindung der Formel (I), in welcher V und W unabhängig voneinander Wasserstoff- oder Halogenatome, Alkylgruppen mit 1 bis 4 Kohlenstoffatomen, Alkoxygruppen mit 1 bis 4 Kohlenstoffatomen, darstellen, Z einen Phenylrest, Thienyl-, Pyridylrest oder einen durch einen oder zwei Substituenten, ausgewählt aus den Halogenatomen, den Alkyl- und Alkoxygruppen mit 1 bis 4 Kohlenstoffatomen, den Trifluormethyl- oder Nitrogruppen, substituierten Phenylrest darstellt, $R_1$ und $R_2$ die obige Bedeutung haben, eine Verbindung der Formel:

$$Hal\text{-}CH_2\text{-}CO\text{-}N{<}^{R_1}_{R_2} \qquad (V)$$

worin $R_1$ und $R_2$ die in der Formel (I) angegebene Bedeutung haben und Hal ein Halogenatom darstellt, mit einer Verbindung der Formel:

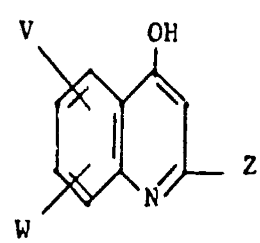

(VI)

in welcher V, W und Z die obige Bedeutung haben,

umsetzt, die Verbindung isoliert und sie gegebenenfalls in ein Säureadditionssalz umwandelt,

c - zur Herstellung einer Verbindung der Formel (I), in welcher V und W unabhängig voneinander Wasserstoff- oder Halogenatome, Alkylgruppen mit 1 bis 4 Kohlenstoffatomen oder Alkoxygruppen mit 1 bis 4 Kohlenstoffatomen darstellen, Z einen Phenyl-, Thienyl-, Pyridylrest oder einen durch einen oder zwei Substituenten, ausgewählt aus den Halogenatomen, den Alkyl- und Alkoxygruppen mit 1 bis 4 Kohlenstoffatomen, den Trifluormethyl- oder Nitrogruppen, substituierten Phenylrest darstellt und $NR_1R_2$ einen durch eine Alkoxygruppe mit 1 bis 4 Kohlenstoffatomen substituierten Piperidinring darstellt, eine Verbindung der Formel:

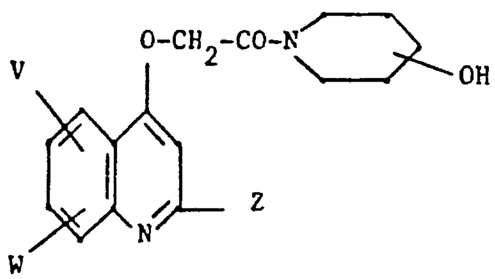

(VII)

mit einer Verbindung der Formel:

Hal-R (VIII)

in welchen V, W und Z die zuvor angegebene Bedeutung haben, Hal ein Halogenatom darstellt und R eine Alkylgruppe mit 1 bis 4 Kohlenstoffatomen darstellt, reagieren läßt, die Verbindung isoliert und sie gegebenenfalls in ein Säureadditionssalz überführt,

d - zur Herstellung einer Verbindung der Formel (I), in welcher V und W unabhängig voneinander Wasserstoff- oder Halogenatome, Alkylgruppen mit 1 bis 4 Kohlenstoffatomen oder Alkoxygruppen mit 1 bis 4 Kohlenstoffatomen darstellen, Z einen Phenyl-, Thienyl-, Pyridylrest oder einen durch einen oder zwei Substituenten, ausgewählt aus den Halogenatomen, den Alkyl- und Alkoxygruppen mit 1 bis 4 Kohlenstoffatomen, den Trifluormethyl- oder Nitrogruppen substituierten Phenylrest darstellt und $NR_1R_2$ einen Piperazinonring darstellt, der am Stickstoffatom durch eine Alkylgruppe mit 1 bis 4 Kohlenstoffatomen substituiert ist, eine entsprechende Verbindung der Formel (I), in welcher $NR_1R_2$ einen Piperazinonring darstellt, mit einer Verbindung der Formel:

Hal-R (VIII)

in welcher Hal ein Halogenatom darstellt und R eine Alkylgruppe mit 1 bis 4 Kohlenstoffatomen darstellt, reagieren läßt, das Produkt isoliert und gegebenenfalls in ein Säureadditionssalz überführt,

e - zur Herstellung einer Verbindung der Formel (I), worin V und/oder W Aminogruppen sind und/oder Z ein durch eine oder zwei Aminogruppen substituierter Phenylrest ist, $R_1$ und $R_2$ die obige Bedeutung haben, wobei diese Verbindungen selbstverständlich keine Nitrogruppe enthalten, die entsprechende Nitroverbindung katalytisch hydriert, das Produkt isoliert und gegebenenfalls in ein Säureadditionssalz überführt,

f - zur Herstellung einer Verbindung der Formel (I), in welcher V und/oder W Aminogruppen sind und/ oder Z ein durch eine oder zwei Aminogruppen substituierter Phenylrest ist, $R_1$ und $R_2$ die obige Bedeutung haben, wobei diese Verbindungen selbstverständlich keine Nitrogruppe enthalten, die entsprechende Nitroverbindung reduziert, das Produkt isoliert und gegebenenfalls in ein Säureadditionssalz überführt,

g - zur Herstellung einer Verbindung der Formel (I), worin V und/oder W Acylaminogruppen mit 1 bis 4 Kohlenstoffatomen sind, Z, $R_1$ und $R_2$ die obige Bedeutung haben, wobei Z selbstverständlich kein durch eine Aminogruppe substituierter Phenylrest ist, die entsprechende Verbindung der Formel (I), worin V und/oder W Aminogruppen sind, mit einem Acylierungsmittel der Formel:

Hal-CO-E (IX)

umsetzt, in welcher Hal ein Halogenatom darstellt und E ein Wasserstoffatom oder eine Alkylgruppe mit 1 bis 3 Kohlenstoffatomen darstellt, das Produkt isoliert und gegebenenfalls in ein Säureadditionssalz überführt.

**Claims for the Contracting States:**
BE, CH, DE, FR, GB, IT, LI, LU, NL, SE

1. A racemic or stereoisomeric compound of formula (I)

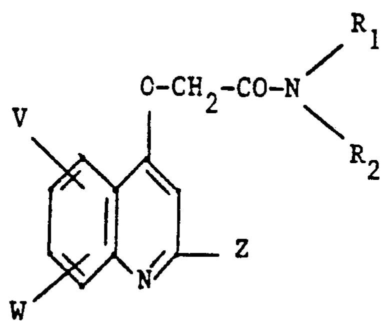

(I)

in which V and W, which may be identical or different, denote hydrogen or halogen atoms, alkyl groups containing 1 to 4 carbon atoms, alkoxy groups containing 1 to 4 carbon atoms, amino groups or acylamino groups containing 1 to 4 carbon atoms,

Z denotes a phenyl, thienyl or pyridyl radical, or a phenyl radical substituted with one or two substituents selected from halogen atoms, alkyl and alkoxy groups containing 1 to 4 carbon atoms, and trifluoromethyl, nitro or amino groups,

$R_1$ denotes a linear or branched alkyl group containing 1 to 6 carbon atoms, an alkoxyalkyl group in which the alkyl and alkoxy portions each contain 1 to 4 carbon atoms, a cycloalkyl group containing 3 to 6 carbon atoms, a phenyl group, a phenylalkyl group in which the alkyl portion contains 1 to 4 carbon atoms or a cycloalkyl group in which the alkyl portion contains 1 to 3 carbon atoms and the cycloalkyl portion contains 3 to 6 carbon atoms,

$R_2$ denotes a linear or branched alkyl group containing 1 to 6 carbon atoms, an alkoxyalkyl group in which the alkoxy and alkyl portions each contain 1 to 4 carbon atoms, a cycloalkyl group containing 3 to 6 carbon atoms, a phenyl group, a phenylalkyl group in which the alkyl portion contains 1 to 4 carbon atoms or a cycloalkyl group in which the alkyl portion contains 1 to 3 carbon atoms and the cycloalkyl portion contains 3 to 6 carbon atoms, or a 4-morpholine ring, and

$R_1$ and $R_2$ can also form, together with the nitrogen atom to which they are attached, a pyrrolidine ring, a piperidine ring optionally substituted with a hydroxy group, an alkyl group containing 1 to 4 carbon atoms, an alkoxy group containing 1 to 4 carbon atoms or an alkyloxycarbonyl group in which the alkyl portion contains 1 to 4 carbon atoms, or morpholine ring optionally substituted with one or two alkyl groups containing 1 to 4 carbon atoms, a thiomorpholine ring, a piperazine ring optionally substituted on the nitrogen atom with an alkyl group containing 1 to 4 carbon atoms, an alkyloxycarbonyl group in which the alkyl portion contains 1 to 4 carbon atoms, an acyl group containing 2 to 5 carbon atoms, or a formyl group, or a piperazinone ring optionally substituted on the nitrogen atom with an alkyl group containing 1 to 4 carbon atoms, and also, where it can exist, a salt of such a compound with an acid.

2. A process for preparing a compound according to claim 1, wherein an amine of formula:

$$HN{<}^{R_1}_{R_2} \qquad (II)$$

in which $R_1$ and $R_2$ have the same meanings as in the formula (I), or a salt of this amine, is reacted with a derivative of formula:

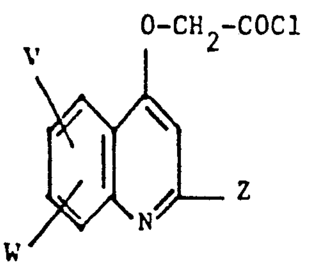

(III)

in which V, W and Z have the same meanings as in the formula (I), and the product is isolated and optionally converted to an addition salt with an acid.

3. A process for preparing a compound according to claim 1, in the formula of which V and W, which may be identical or different, denote hydrogen or halogen atoms, alkyl groups containing 1 to 4 carbon atoms or alkoxy groups containing 1 to 4 carbon atoms, Z denotes a phenyl, thienyl or pyridyl radical or a phenyl radical substituted with one or two substituents chosen from halogen atoms, alkyl and alkoxy groups containing 1 to 4 carbon atoms, and trifluoromethyl or nitro groups, and $R_1$ and $R_2$ are defined as in claim 1, wherein a compound of formula:

$$Hal\text{-}CH_2\text{-}CO\text{-}N{<}^{R_1}_{R_2} \qquad (V)$$

in which $R_1$ and $R_2$ are defined as in the formula (I) and Hal denotes a halogen atom, is reacted with a derivative of formula:

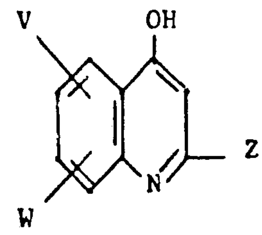

(VI)

in which V, W and Z are defined as above, and the product is isolated and optionally converted to an addition salt with an acid.

4. A process for preparing a compound according to claim 1, in the formula of which V and W, which may be identical or different, denote hydrogen or halogen atoms, alkyl groups containing 1 to 4 carbon atoms or alkoxy groups containing 1 to 4 carbon atoms, Z denotes a phenyl, thienyl or pyridyl radical or a phenyl radical substituted with one or two substituents chosen from halogen atoms, alkyl and alkoxy groups containing 1 to 4 carbon atoms, and trifluoromethyl or nitro groups and $NR_1R_2$ denotes a piperidine ring substituted with an alkoxy group containing 1 to 4 carbon atoms, wherein a compound of formula:

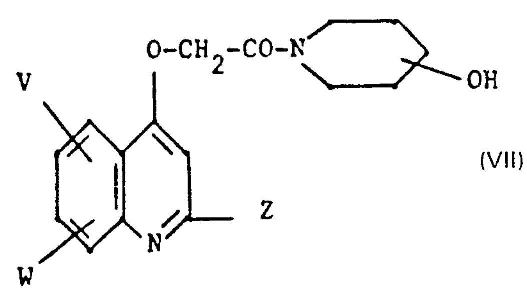

(VII)

is reacted with a derivative of formula:

$$Hal\text{-}R \qquad (VIII)$$

in which V, W and Z are defined as above, Hal denotes a halogen atom and R denotes an alkyl group containing 1 to 4 carbon atoms, and the product is isolated and optionally converted to an addition salt with an acid.

5. A process for preparing a compound according to claim 1, in the formula of which V and W, which may be identical or different, denote hydrogen or halogen atoms, alkyl groups containing 1 to 4 carbon atoms or alkoxy groups containing 1 to 4 carbon atoms, Z denotes a phenyl, thienyl or pyridyl radical or a phenyl radical substituted with one or two substituents chosen from halogen atoms, alkyl and alkoxy groups containing 1 to 4 carbon atoms, and trifluoromethyl or nitro groups and $NR_1R_2$ denotes a piperazinone ring substituted on the nitrogen atom with an alkyl group containing 1 to 4 carbon atoms,

wherein a corresponding compound of formula (I) in which $NR_1R_2$ denotes a piperazinone ring is reacted with an alkyl halide of formula Hal-R (VIII), in which Hal denotes a halogen atom and R denotes an alkyl group containing 1 to 4 carbon atoms, and the product is isolated and optionally converted to an addition salt with an acid.

6. A process for preparing a compound according to claim 1, in the formula of which V and/or W are amino groups and/or Z is a phenyl radical substituted with one or two amino groups, and $R_1$ and $R_2$ are defined as in claim 1, with the proviso that these compounds are devoid of nitro groups, wherein the corresponding nitro derivative is catalytically hydrogenated and the product is isolated and optionally converted to an addition salt with an acid.

7. A process for preparing a compound according to claim 1, in the formula of which V and/or W are amino groups and/or Z is a phenyl radical substituted with one or two amino groups, and $R_1$ and $R_2$ are defined as in claim 1, with the proviso that these compounds are devoid of nitro groups, wherein the corresponding nitro derivative is reduced and the product is isolated and optionally converted to an addition salt with an acid.

8. A process for preparing a compound according to claim 1, in the formula of which V and/or W are acylamino groups containing 1 to 4 carbon atoms, and Z, $R_1$ and $R_2$ are defined as in claim 1, with the proviso that Z is not a phenyl radical substituted with an amino group, wherein the corresponding compound of formula (I) for which V and/or W are amino groups is reacted with an acylating agent of formula:

Hal-CO-E (IX)

in which Hal denotes a halogen atom and E denotes a hydrogen atom or an alkyl group having 1 to 3 carbon atoms, and the product is isolated and optionally converted to an addition salt with an acid.

9. A drug containing at least one active substance and optionally one or more diluents or adjuvants which are compatible and pharmaceutically acceptable, in which drug the active substance is a compound according to claim 1.

**Claim for the Contracting State:** AT

1. A process for preparing a racemic or stereoisomeric compound of formula (I)

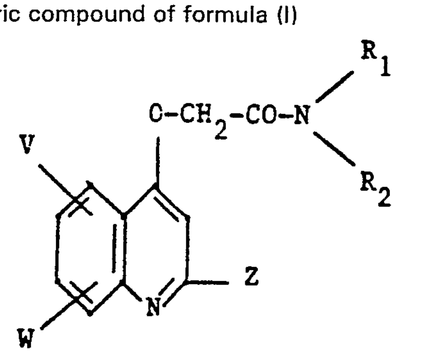

(I)

in which V and W, which may be identical or different, denote hydrogen or halogen atoms, alkyl groups containing 1 to 4 carbon atoms, alkoxy groups containing 1 to 4 carbon atoms, amino groups or acylamino groups containing 1 to 4 carbon atoms,

Z denotes a phenyl, thienyl or pyridyl radical, or a phenyl radical substituted with one or two substituents selected from halogen atoms, alkyl or alkoxy groups containing 1 to 4 carbon atoms, and trifluoromethyl, nitro or amino groups,

$R_1$ denotes a linear or branched alkyl group containing 1 to 6 carbon atoms, an alkoxyalkyl group in which the alkyl and alkoxy portions each contain 1 to 4 carbon atoms, a cycloalkyl group containing 3 to 6 carbon atoms, a phenyl group, a phenylalkyl group in which the alkyl portion contains 1 to 4 carbon atoms or a cycloalkyl group in which the alkyl portion contains 1 to 3 carbon atoms and the cycloalkyl portion contains 3 to 6 carbon atoms,

$R_2$ denotes a linear or branched alkyl group containing 1 to 6 carbon atoms, an alkoxyalkyl group in which the alkoxy and alkyl portions each contain 1 to 4 carbon atoms, a cycloalkyl group containing 3 to 6 carbon atoms, a phenyl group, a phenylalkyl group in which the alkyl portion contains 1 to 4 carbon atoms or a cycloalkyl group in which the alkyl portion contains 1 to 3 carbon atoms and the cycloalkyl portion contains 3 to 6 carbon atoms, or a 4-morpholine ring, and

$R_1$ and $R_2$ can also form, together with the nitrogen atom to which they are attached, a pyrrolidine ring, a piperidine ring optionally substituted with a hydroxy group, an alkyl group containing 1 to 4 carbon atoms, an alkoxy group containing 1 to 4 carbon atoms or an alkyloxycarbonyl group in which the alkyl portion contains 1 to 4 carbon atoms, or morpholine ring optionally substituted with one or two alkyl groups containing 1 to 4 carbon atoms, a thiomorpholine ring, a piperazine ring optionally substituted on the nitrogen atom with an alkyl group containing 1 to 4 carbon atoms, an alkyloxycarbonyl group in which the alkyl portion contains 1 to 4 carbon atoms, an acyl group containing 2 to 5 carbon atoms, or a formyl group, or a piperazinone ring optionally substituted on the nitrogen atom with an alkyl group containing 1 to 4 carbon atoms, and also, where it can exist, a salt of such a compound with an acid, wherein:

a - for preparing a compound of formula (I) in which V, W, Z, $R_1$ and $R_2$ have all the meanings stated above, an amine of formula:

$$HN\begin{smallmatrix}R_1\\R_2\end{smallmatrix} \qquad (II)$$

in which $R_1$ and $R_2$ have the same meanings as in the formula (I), or a salt of this amine, is reacted with a derivative of formula:

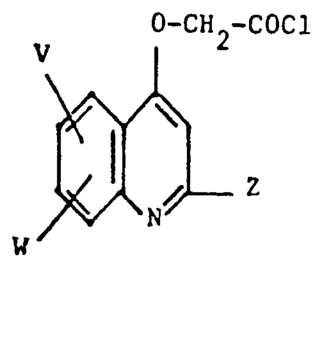

(III)

in which V, W and Z have the same meanings as in the formula (I), and the product is isolated and optionally converted to an addition salt with an acid,

b - for preparing a compound of formula (I) in which V and W, which may be identical or different, denote hydrogen or halogen atoms, alkyl groups containing 1 to 4 carbon atoms or alkoxy groups containing 1 to 4 carbon atoms, Z denotes a phenyl, thienyl or pyridyl radical or a phenyl radical substituted with one or two substituents chosen from halogen atoms, alkyl and alkoxy groups containing 1 to 4 carbon atoms, and trifluoromethyl or nitro groups, and $R_1$ and $R_2$ are defined as above, a compound of formula:

$$Hal\text{-}CH_2\text{-}CO\text{-}N{<}^{R_1}_{R_2} \qquad (V)$$

in which $R_1$ and $R_2$ are defined as in the formula (I) and Hal denotes a halogen atom, is reacted with a derivative of formula:

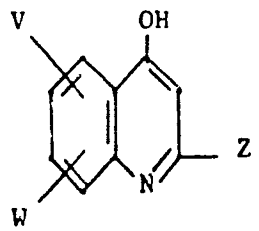

(VI)

in which V, W and Z are defined as above, and the product is isolated and optionally converted to an addition salt with an acid,

c - for preparing a compound of formula (I) in which V and W, which may be identical or different, denote hydrogen or halogen atoms, alkyl groups containing 1 to 4 carbon atoms or alkoxy groups containing 1 to 4 carbon atoms, Z denotes a phenyl, thienyl or pyridyl radical or a phenyl radical substituted with one or two substituents chosen from halogen atoms, alkyl and alkoxy groups containing 1 to 4 carbon atoms, and trifluoromethyl or nitro groups and $NR_1R_2$ denotes a piperidine ring substituted with an alkoxy group containing 1 to 4 carbon atoms, a compound of formula:

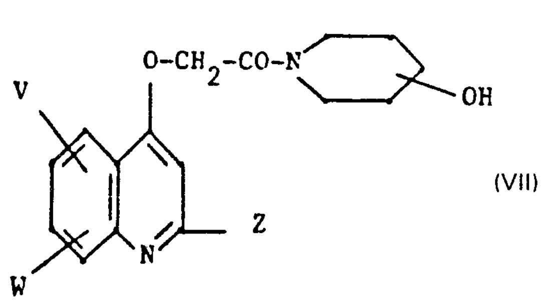

(VII)

is reacted with a derivative of formula:

Hal-R (VIII)

in which V, W and Z are defined as above, Hal denotes a halogen atom and R denotes an alkyl group containing 1 to 4 carbon atoms, and the product is isolated and optionally converted to an addition salt with an acid,

d - for preparing a compound of formula (I) in which V and W, which may be identical or different, denote hydrogen or halogen atoms, alkyl groups containing 1 to 4 carbon atoms or alkoxy groups containing 1 to 4 carbon atoms, Z denotes a phenyl, thienyl or pyridyl radical or a phenyl radical substituted with one or two substituents chosen from halogen atoms, alkyl and alkoxy groups containing 1 to 4 carbon atoms, and trifluoromethyl or nitro groups and $NR_1R_2$ denotes a piperazinone ring substituted on the nitrogen atom with an alkyl group containing 1 to 4 carbon atoms, a corresponding compound of formula (I) in which $NR_1R_2$ denotes a piperazinone ring is reacted with a derivative of formula:

Hal-R (VIII)

in which Hal denotes a halogen atom and R denotes an alkyl group containing 1 to 4 carbon atoms, and the product is isolated and optionally converted to an addition salt with an acid,

e - for preparing a compound of formula (I) in which V and/or W are amino groups and/or Z is a phenyl radical substituted with one or two amino groups, and $R_1$ and $R_2$ are defined as above, with the proviso that these compounds are devoid of nitro groups, the corresponding nitro derivative is catalytically hydrogenated and the product is isolated and optionally converted to an addition salt with an acid,

f - for preparing a compound of formula (I) in which V and/or W are amino groups and/or Z is a phenyl radical substituted with one or two amino groups, and $R_1$ and $R_2$ are defined as above, with the proviso that these compounds are devoid of nitro groups, the corresponding nitro derivative is reduced and the product is isolated and optionally converted to an addition salt with an acid,

g - for preparing a compound of formula (I) in which V and/or W are acylamino groups containing 1 to 4 carbon atoms, and Z, $R_1$ and $R_2$ are defined as above, with the proviso that Z is not a phenyl radical substituted with an amino group, the corresponding compound of formula (I) for which V and/or W are amino groups is reacted with an acylating agent of formula:

Hal-CO-E (IX)

in which Hal denotes a halogen atom and E denotes a hydrogen atom or an alkyl group having 1 to 3 carbon atoms, and the product is isolated and optionally converted to an addition salt with an acid.